# EUROPEAN PATENT APPLICATION

(11) **EP 4 593 024 A1**
(43) Date of publication of application: **30.07.2025**
(21) Application number: 25154363.3
(22) Date of filing: 28.01.2025
(51) Int. Cl.: G16H 20/70

(54) **DYNAMICALLY TARGETING SUBSTANCE USE DISORDERS AND CONDITIONS VIA PERSONALIZED DIGITAL THERAPEUTICS**

(30) Priority: 29.01.2024 US 202463626465 P; 18.11.2024 US 202418951081
(71) Applicant: Click Therapeutics, Inc., New York, NY 10013 (US)
(72) Inventor: Adler, Samantha, New York, 10013 (US); Forster, Sarah, New York, 10013 (US)
(74) Representative: Marks & Clerk LLP

(57) **Abstract**

Provided herein are systems and methods for presenting interventions to address substance use disorders (SUDs) in users. One or more processors may identify for a user with a substance use disorder, a profile including a plurality of substance use words, memory words, and neutral words. The one or more processors may provide a repeated cycle of selection activities and visualization activities. The one or more processors may update the profile of the user using the performance of the user in the activity. The systems and methods provided herein can enhance the efficacy of the medication that the user is taking in concurrence with addressing the SUD.

## Description

### CROSS-REFERENCE TO RELATED APPLICATIONS

The present application claims priority to and the benefit of U.S. Provisional Patent Application No. 63/626,465, titled "MANAGING SESSION TO PROVIDE PSYCHOTHERAPEUTIC COMPONENTS TO ADDRESS USER CONDITIONS", and filed on January 29, 2024, the entire contents of which are hereby incorporated by reference in entirety.

### BACKGROUND

Substance use disorders (SUDs) arise due to recurrent, compulsive use of alcohol, drugs, or other such substances leading to cognitive impairment, health risks, and disability. Common SUDs include alcohol use disorder (AUD), opioid use disorder (OUD), or drug use disorder, among others. This condition arises from a diverse array of factors such as biological and environmental factors. For example, specific factors that can lead to SUDs include genetics, brain chemistry (e.g., changes in neurotransmitter levels like dopamine), mental health disorders (e.g., anxiety, depression, or attention deficit hyperactivity disorder (ADHD)), personality traits (e.g., impulsive behavior), coping mechanisms (e.g., lacking effective coping strategies), or early exposure (e.g., during adolescence when the brain is still developing), among others. Other factors that can lead to SUDs include outside influences such as family (e.g., substance use in family), peer pressure (e.g., peers initiating substance use), availability (e.g., accessibility to substances), socioeconomic status (e.g., poverty, unemployment), cultural norms (e.g., cultures with an emphasis on alcohol), or media (e.g., media portrayals of substance use), among others.

SUDs impact the physical and mental health, social well-being, and overall quality of life of individuals affected. Individuals with SUDs are at an increased susceptibility for a variety of physical health complications. Additionally, those having SUDs are at a greater risk of psychological disorders such as cognitive impairment and emotional distress as well as heightened risk for drug overdose. The overall quality of life for those with SUDs is often severely compromised.

At the nervous system level, SUDs lead to changes in synaptic strength and connectivity, particularly in the brain's reward circuitry, contributing to the persistence of addictive behaviors. Specifically, the SUD alters the brain structure and function as well as leading to imbalances in neurotransmitters which are essential for cognition and overall brain function. Furthermore, individuals with SUDs are at a heightened risk for developing neurological disorders (e.g., stroke, Parkinson's disease) and mental health disorders (e.g., depression, anxiety). For example, opioids bind to opioid receptors in the brain which reduce perception of pain and induces euphoria. Specifically, developing an SUD such as OUD leads to impairments in cognitive function (e.g., executive function, memory) and alters the brain structure, specifically to areas related to stress, decision-making, and behavior regulation.

Treatments for SUDs can be used to stop and reduce side effects of substance use. These treatments include, for example, in-person behavioral therapies, medication, in- and outpatient treatment programs, detoxification, or support groups, among others. Mitigating relapses of SUDs, however, is difficult due to ineffective treatments and accessibility. In particular, an individual's motivation to adhere to the treatments as well as failure to address underlying cognitive impairment of the individual leading to substance use reduces the effectiveness of SUD treatments. For example, an individual participating in inpatient rehabilitation may experience relapse once transitioning back to daily life. Failing to take into account both cognitive impairment and psychological components (e.g., motivation) can lead to ineffective SUD treatments and ultimately little to no improvement in the substance use disorder, and potentially relapse. These treatments do not take into account challenges associated with maintaining an individual's commitment to refrain from substance use as well as the cognitive impact of the SUD on the individual. Furthermore, these treatments are not directly targeted at rewiring or modulating the components of the nervous system that contribute to persistence of maladaptive behaviors associated with SUD.

### SUMMARY

Presented herein are systems and methods of providing digital therapeutics to target users' executive control systems. The digital therapeutic application described herein delivers a unique combination of multi-component neuromodulatory interventions aimed at targeting cognitive defects associated with substance use disorder (SUD). Specifically, this novel integrated approach first combines two training activities in a single, immersive challenge, to target overlapping neural network substrates, which functions to train two subdomains of executive control (e.g., inhibitory control and working memory) simultaneously, potentially simulating a critical-period-like state to enhance plasticity by a flexibility mechanism. The combined training paradigm adds a third training activity as a synergistic psychotherapeutic component that can capitalize on the plasticity induced by the prior two activities, by applying the third training to an activity relevant to the real world, thus helping to guide real life behavior that is relevant to the substance use disorder that the user is intending to target, leading to an enhanced clinical impact of these exercises.

The maximum benefit is obtained by continuously performing these three training activities together in repeated cycles, particularly in a combined training program. Over time, the user's habit-based drug-taking responses can transition into responses focused on achieving future goals. Thus, the first two training activities build the user's "mental muscles" that control impulses and making plans, while the third training activity applies the skills built up during the first two training activities in real world situations. The combination of these three training modules has a synergistic effect in strengthening the user's impulse control to combat substance use disorder.

The integration is a marked improvement over other intervention techniques that focus on only cognitive components or psychological components, not the integration of the two nor overcoming limits in transferring effects to real world conditions. If there is an imbalance represented between an overactive impulsivity system and an underactive executive control system, the training activities disclosed herein can improve capacity and efficiency of the executive control system to improve functionality. The combination of the three training activities described herein targets overlapping neural network substrates within the prefrontal cortex, involved in both decision-making and working memory functions with the potential to optimize the brain's learning mechanisms.

In addition, the digital therapeutics application addresses the lack of personalized solutions that effectively target both decision making and working memory functions of the brain. The application blends activities targeting inhibitory control and working memory simultaneously, while also integrating goal-related context to translate improvements in inhibitory control and working memory to real-world improvements. Based on a performance of the user on the activities, the application increases the difficulty of the activities to further improve inhibitory control and working memory in the individual. The digital therapeutic application is able to use information on the user to specifically tailor interventions for the SUD. Scores of the results of activities indicate a performance and progress of the user, and can be presented to the user to encourage the user to adhere with the intervention regimen as they are able to see their progress. By continuously updating and challenging the user during the intervention, the digital therapeutic application ensures that the intervention remains effective and aligned with the evolving state of the user's executive control system.

Accordingly, the digital therapeutic application addresses the lack of integrated cognitive and psychological treatment by providing digital interventions that dynamically adjust based on individual responses to various activities. The application further offers a tailored approach to addressing SUDs that align with specific needs and physiological profiles of each user. Through this integration of digital, cognitive, and psychological solutions, the efficacy of the treatment of SUDs and related disorders is greatly improved, leading to better outcomes and overall patient care.

SUDs present several challenges impacting physical health, social well-being, and mental health of individuals affected. Individuals with SUDs are at an increased susceptibility for a variety of physical health complications, including, liver disease, lung disease, cardiovascular disease, neurological damage, human immunodeficiency viruses (HIV), hepatitis B and C, sexually transmitted infections (STDs), nutritional deficiencies, sleep disorders, and a heightened risk for overdose.

The digital therapeutic application described herein creates and maintains for each user a set of substance use words associated with the user's SUD and a set of memory words not related to substance use (e.g., different from the set of substance use words).

During a first part, the user is instructed not to select substance use words or memory words that may be presented to the user and instead to only select neutral words. This activity improves the executive control system function within the user such as by breaking associations (e.g., triggers) the user has with substance use. Over time, the substance use words and memory words (meaning, words that the user should withhold a response to) can appear less frequently than the neutral words (meaning, words that the user should respond to), allowing the latter to become prepotent (e.g., automatic). This also teaches the user to give less attention to the substance use words. Further, because the words appear for a short time for the user to respond to (or not respond to), this encourages rapid response rates to train inhibitory control and to break connections the user may have with the substance use words.

During a second part, the user is instructed to perform an activity based on visualizing a future event. The future event may be related to the user's health, relationships, values, finances, enjoyment, charitable contributions, sobriety, lifestyle, overcoming maladaptive behavior associated with the substance use of the user, or some other category. Alternatively, the user may be instructed to create or identify a goal to accomplish. The user is then instructed to create a cue word or image to remind the user of the future event at later sessions.

During a third part, the user is instructed to perform an activity similar to the first part, specifically to not select substance use words or memory words that appear on the screen and instead to select neutral words. During a fourth part, the user is shown a prompt that contains the previously-generated cue word or image so the user can visualize the future event again. The prompt can be a description or visual depiction of the future event or even a vision board simulating sticky notes or hand-written notes taped on a cork board, showing information from multiple sessions (different future events) collected into one design or screen. The user has the option to modify, remove, or create new cue words or images over time (e.g. as goals change over time, or as the time for the future event passes).

This sequence may repeat in a cycle many times over time: (1) asking the user not to select substance use words and/or memory words that appear on the screen then (2) asking the user to visualize future events based on previously-generated cue words or images, including creating new future events and new cue words or images. In this manner, the application may use various activities provided at various times to improve executive control to reduce substance use and mitigate relapse in the user, such periodic activities tailored towards a progress of the user by improving inhibitory control and working memory while also capitalizing on plasticity induced by improvement in executive control via visualizing future events. This application can blend various techniques to target depreciated function of the brain by substance use while providing sustainable interventions to promote adherence by the user while avoiding unnecessary time and money spent on conventional SUD treatments that do not work.

The application provides for adaptive difficulty of the activities to continually stimulate and challenge the user as the user becomes more skilled and experienced with the activities, further strengthening the user's executive control. For example, over time, new memory words can be added to the list for the user to remember and withhold responding to. This can become progressively difficult, such as more words being added per day. The application can also provide a quiz to test the user on their memory of all the memory words, and the results of the quiz used to determine whether to increase the number of memory words or instead keep the number the same. As another example, by creating a score for the user based on the number or percentage of correct interactions with the substance use words, memory words, or both; a speed of user selections, or both. Once the score satisfies a threshold, the application may increase in difficulty by, for example, increasing a number of words for the subsequent activity.

The digital therapeutic application described herein can reopen a window of plasticity in adults, typically limited to childhood. The digital therapeutic can mimic key principles of learning observed during time periods in which an experience shapes a trait and behaviors are developed (e.g., plasticity). For example, the first training activities with prepotent responses, short response times, and indication-specific words can create a challenging and salient learning environment for the user to develop rapid adaptations under the conditions and focused attention. Further, the progressive difficulty element of the second training mirrors how skills are naturally acquired during developmental sensitive periods (e.g., plasticity periods in childhood). The third training activities can leverage neural plasticity which underpins the formation of episodic memories throughout a lifespan of the user. Combining the third training with both the first and second trainings supports neural plasticity across associated brain regions and can enhance a clinical impact of the digital therapeutic application.

### BRIEF DESCRIPTION OF THE DRAWINGS

The foregoing and other objects, aspects, features, and advantages of the disclosure will become more apparent and better understood by referring to the following description taken in conjunction with the accompanying drawings, in which:
FIG. 1 depicts a block diagram of a system for presenting interactive sessions to address substance use in users in accordance with an illustrative embodiment.
FIGs. 2A and 2B depict a block diagram for a process to provide a session to a user to perform a substance use word activity, in accordance with an illustrative embodiment.
FIGs. 3A and 3B depict a block diagram for a process to provide a session to a user to perform a visualization activity, in accordance with an illustrative embodiment.
FIGs. 4A and 4B depict a block diagram for a process to provide a session to a user to perform a memory quiz activity, in accordance with an illustrative embodiment.
FIG. 5 depicts a flow diagram for a visualization activity, in accordance with an illustrative embodiment.
FIG. 6 depicts a flow diagram of a method for selecting activities for the user to address substance use in accordance with an illustrative embodiment.
FIG. 7 illustrates an example study schedule of an engagement study for the CT-102 mobile app.
FIG. 8 depicts a flow diagram of a method of ameliorating substance use in users via administration of digital therapeutics in accordance with an illustrative embodiment.
FIG. 9 is a block diagram of a server system and a client computer system in accordance with an illustrative embodiment.

### DETAILED DESCRIPTION

For purposes of reading the description of the various embodiments below, the following enumeration of the sections of the specification and their respective contents may be helpful:

Section A describes systems and methods for providing instructions for an intervention for a substance use disorder (SUD) in a user.

Section B describes systems and methods for ameliorating substance use in users via administration of digital therapeutics.

Section C describes a network and computing environment which may be useful for practicing embodiments described herein.

### A. Systems and Methods for Providing Instructions for an Intervention for a SUD in a User

Aspects of the present disclosure are directed towards systems and methods for selecting treatments for providing instructions for activities to address maladaptive behaviors associated with substance use in users. One or more processors can identify, for a user with a substance use disorder, a profile including a plurality of substance use words. The one or more processors can present a plurality of memory words not related to the substance use, and an instruction to memorize the plurality of memory words. The one or more processors can provide an instruction to perform an activity to refrain from selecting one or more of the plurality of memory words and/or one or more of the plurality of substance use words. The one or more processors can update the profile of the user using the performance of the user in the activity. The activity to refrain from selecting one or more of the plurality of memory words can be performed concurrently with the activity to refrain from selecting one or more of the plurality of substance use words.

In various implementations, the one or more processors can provide an instruction to perform an activity to provide information on a future event. The future event can be related to at least one of the user's health, relationships, values, finances, enjoyment, charitable contributions, sobriety, small steps, achievements, future gratitude, challenges, lifestyle, or overcoming maladaptive behavior associated with substance use. The one or more processors can provide, prior to providing the instruction to perform the activity, an instruction for the user to select a type of future event selected from at least one of the user's health, relationships, values, finances, enjoyment, charitable contributions, sobriety, small steps, achievement, future gratitude, challenges, lifestyle, or overcoming maladaptive behavior associated with substance use. The one or more processors can also provide, prior to the instruction to perform the activity, an instruction for the user to select a category of the future event. The category of the future event can be selected from the user's health, relationships, values, finances, enjoyment, charitable contributions, sobriety, small steps, achievement, future gratitude, challenges, lifestyle, or overcoming maladaptive behavior associated with substance use.

In various implementations, the one or more processors can provide an instruction to select a timeframe for the future event. The future event may occur in less than a month. The future event may occur in more than a month. The one or more processors can instruct the user to generate one or more visual cues based on the information on the future event provided by the user. The visual cue can be an image or text, including free text provided by the user. The one or more processors can provide a subsequent instruction to perform an activity to refrain from selecting one or more of the plurality of memory words and/or one or more of the plurality of substance use words. The instruction may also include an instruction to perform an activity to select one or more neutral words that are unrelated to substance use and different than the memory words. The one or more processors can also provide a prompt containing the visual cue related to the information on the future event and an instruction to perform an activity related to the visual cue. The prompt can be a timeline identifying more than one visual cues. The timeline can automatically update based on the time tagged to the visual cue. The activity can include an audio recording or a textual description.

In some aspects, the activity includes providing information related overcoming maladaptive behaviors associated with substance use or providing information related to the user's health, relationships, values, finances, enjoyment, charitable contributions, sobriety, or lifestyle. In some aspects, an instruction for the user to select an activity selected from providing information related overcoming maladaptive behaviors associated with substance use or providing information related to the user's health, relationships, values, finances, enjoyment, charitable contributions, sobriety, or lifestyle is provided prior to providing the instruction to perform the activity. In some other aspects, an instruction for the user to select a category of the future event is provided prior to the instruction to perform the activity.

In various implementations, the one or more processors can provide a subsequent instruction to perform an activity to refrain from selecting one or more of the plurality of memory words and/or one or more of the plurality of substance use words. The one or more processors can provide, following performance of the activity to refrain from selecting one or more of the plurality of memory words and presentation of one or more of the plurality of substance use words, the prompt related to the information on the future event. The one or more processors can also provide, prior to the instructions to perform an activity to refrain from selecting one or more of the plurality of memory words and one or more of the plurality of substance use words, an instruction to perform an activity to refrain from selecting one or more of the plurality of substance use words. Similarly, the instruction may include an instruction to perform an activity to select one or more neutral words that are not related to substance use and are not one or more of the memory words.

In various implementations, the one or more processors can provide, an instruction to perform an activity to select all of the memory words from a plurality of words. The one or more processors can provide, an instruction to perform an activity to select one or more of the memory words from a plurality of words. The one or more processors can determine a score indicating a percentage of correct interactions with the memory words and then selecting, by the one or more processors, the plurality of memory words based on the score. In various implementations, a correct interaction may refer to the user selecting the word (e.g.: selecting, engaging, choosing, contacting, or touching) or refraining from selecting the word (e.g.: allowing the word to be displayed without choosing, engaging with, contacting, or touching the word on the display).

In various implementations, the one or more processors can determine a score indicating correct interactions, speed of the user selections, or both during performance of the activity to select all memory words from a plurality of words. The one or more processors can determine, responsive to the score satisfying a threshold, an increase in a number of words for a subsequent activity to refrain from selecting one or more of a plurality of memory words, an activity to refrain from selecting one or more of a plurality of memory words, or an activity to select all memory words from a plurality of words. The one or more processors can display the score indicating at least one of a percentage of correct interactions, speed of user selections, or both during one or more activities.

The one or more processors can determine a score indicating correct interactions, speed of user selections, or both during performance of one or more activities and then selecting, by the one or more processors, a subsequent plurality of substance use words or a subsequent plurality of memory words, or both based on the score. The score can be determined by the number of correct interactions, speed of user selections, or both during performance of the activity to select all the memory words from a plurality of words. The one or more processors can determine a score indicating correct interactions, speed of the user selections, or both during performance of the activity to refrain from selecting the one or more of the plurality of memory words and/or the plurality of substance use words. The one or more processors can determine, responsive to the score satisfying a threshold, an increase in a number of words for a subsequent activity to refrain from selecting the plurality of memory words and/or the plurality of substance use words.

The one or more processors can generate, one or more cue words or images based on the information on the future event provided by the user where the prompt related to the information on the future event comprises the one or more cue words or images.

In various implementations, the one or more processors can provide an instruction to perform the activity to refrain from selecting one or more of the plurality of substance use words and/or the one or more of the plurality of memory words can further include providing, by the one or more processors, an instruction to perform an activity for the user to select one or more of a plurality of neutral words unrelated to substance use and different than the memory words. The one or more processors can provide the instruction to perform an activity to refrain from selecting one or more of the plurality of substance use words or memory words comprises presenting the one or more of the plurality of words for less than 1 second, less than 2 seconds, less than 3 seconds, 5 seconds, or equal to or greater than 10 seconds. The instruction to perform an activity can be provided to the user by the one or more processors at least once a day, at least once every 2 days, at least once every 5 days, or at least once a week. The one or more activities can be performed over a period of 1 minute, 2 minutes, 3 minutes, 5 minutes, 7 minutes, 10 minutes or longer. The prompt can be provided, by the one or more processors, at least 1 day, 5 days, 1 week, or one month following the providing of the instruction to perform an activity to provide information on a future event.

The one or more processors can receive a request from the user indicating an onset of the behavior associated with the substance use and initiating, by the one or more processors, responsive to receiving the request, the instructions for activities to address maladaptive behaviors associated with substance use in the user. The one or more processors can determine a time at which to provide the instruction to perform the activity to refrain from selecting one or more of the plurality of memory words and/or the plurality of substance use words where providing the instruction, by the one or more processors, to perform the activity to refrain from selecting one or more of the plurality of substance use words and/or the plurality of memory words further comprises providing the instruction at the determined time.

In various implementations, the user can be administered an effective amount of a medication comprising at least one of naloxone, naltrexone, nalmefene, samidorphan, nalorpine, levallorphan, nalodeine, alvimopan, methylnatrexone, naloxegol, acamprosate, disulfiram, topiramate, gabapentin, methadone, buprenorphine, or lofexidine, or a GLP-1 agonist. In various implementations, the substance use includes at least one of alcohol abuse disorder (AUD), opioid abuse disorder (OUD), or drug abuse.

Another aspect of the present disclosure is directed towards systems including one or more processors for providing instructions for activities to address maladaptive behaviors associated with substance use in users. One or more processors can identify, for a user with a substance use disorder, a profile including a plurality of substance use words. The one or more processors can present a plurality of memory words not related to the substance use, and an instruction to memorize the plurality of memory words. The one or more processors can also provide an instruction to perform an activity to refrain from selecting one or more of the plurality of memory words and/or one or more of the plurality of substance use words. The one or more processors can update the profile of the user using the performance of the user in the activity.

In various implementations, the one or more processors can provide an instruction to perform an activity to provide information on a future event. The future event can be related to at least one of the user's health, relationships, values, finances, enjoyment, charitable contributions, sobriety, small steps, achievement, future gratitude, challenges, lifestyle, or overcoming maladaptive behavior associated with substance use. The one or more processors can provide prior to providing the instruction to perform the activity, an instruction for the user to select a type of future event selected from at least one of the user's health, relationships, values, finances, enjoyment, charitable contributions, sobriety, small steps, achievement, future gratitude, challenges, lifestyle, or overcoming maladaptive behavior associated with substance use. The one or more processors can also provide, prior to the instruction to perform the activity, an instruction for the user to select a category of the future event. The category of the future event can be selected from the user's health, relationships, values, finances, enjoyment, charitable contributions, sobriety, small steps, achievement, future gratitude, challenges, lifestyle, or overcoming maladaptive behavior associated with substance use.

In various implementations, the one or more processors can provide an instruction to select a timeframe for the future event. The future event may occur in less than a month. The future event may occur in more than a month. The one or more processors can instruct the user to generate one or more visual cues based on the information on the future event provided by the user. The visual cue can be an image or text, including free text provided by the user. The one or more processors can provide a subsequent instruction to perform an activity to refrain from selecting one or more of the plurality of memory words and/or one or more of the plurality of substance use words and provide a prompt containing the visual cue related to the information on the future event and an instruction to perform an activity related to the visual cue. The prompt can be a timeline identifying more than one visual cues. The timeline can automatically update based on the time tagged to the visual cue. The activity can include an audio recording or a textual description. The one or more processors can provide following performance of the activity to provide information, a prompt related to the information on the future event. The activity to refrain from selecting one or more of the plurality of memory words can be performed concurrently with the activity to refrain from selecting one or more of the plurality of substance use words.

In various implementations, the one or more processors provide a subsequent instruction to perform an activity to refrain from selecting one or more of the plurality of memory words and/or to refrain from selecting one or more of the plurality of substance use words. The one or more processors can also provide a subsequent instruction to perform an activity to refrain from selecting one or more of the plurality of memory words and/or one or more of the plurality of substance use words and provide, following performance of the activity to refrain from selecting one or more of the plurality of memory words and presentation of one or more of the plurality of substance use words, the prompt related to the information on the future event. The one or more processors can provide an instruction to perform an activity to select all of the memory words from a plurality of words. The one or more processors can provide an instruction to perform an activity to select one or more of the memory words from a plurality of words. The one or more processors can determine a score indicating a percentage of correct interactions with the memory words and select the plurality of memory words based on the score.

In various implementations, the one or more processors can determine a score indicating correct interactions, speed of user selections, or both during performance of one or more activities and select a subsequent plurality of substance use words or a subsequent plurality of memory words, or both based on the score. The score can be determined by the number of correct interactions, speed of user selections, or both during performance of the activity to select all the memory words from a plurality of words. The one or more processors can determine a score indicating correct interactions, speed of the user selections, or both during performance of the activity to refrain from selecting the plurality of memory words and/or the plurality of substance use words and determine responsive to the score satisfying a threshold, an increase in a number of words for a subsequent activity to refrain from selecting the plurality of memory words and/or the plurality of substance use words. The one or more processors can determine a score indicating correct interactions, speed of the user selections, or both during performance of the activity to select all memory words from a plurality of words and determine, responsive to the score satisfying a threshold, an increase in a number of words for a subsequent activity to refrain from selecting a plurality of memory words, an activity to refrain from selecting a plurality of substance use words, or an activity to select all memory words from a plurality of words.

In various implementations, the one or more processors display the score indicating at least one of a percentage of correct interactions, speed of user selections, or both during one or more activities. The one or more processors can generate one or more cue words or images based on the information on the future event provided by the user where the prompt related to the information on the future event comprises the one or more cue words or images. Providing the instruction to perform the activity to refrain from selecting one or more of the plurality of substance use words and/or the one or more of the plurality of memory words can further include the one or more processors to provide an instruction to perform an activity for the user to select one or more of a plurality of neutral words unrelated to substance use and different than the memory words. Providing the instruction to perform an activity to refrain from selecting one or more of the plurality of substance use words or memory words can include presenting the one or more of the plurality of words for less than 1 second, less than 2 seconds, less than 3 seconds, 5 seconds, or equal to or greater than 10 seconds.

In various implementations, the instruction to perform an activity is provided to the user at least once a day, at least once every 2 days, at least once every 5 days, or at least once a week. The user can be administered an effective amount of a medication comprising at least one of naloxone, naltrexone, nalmefene, samidorphan, nalorpine, levallorphan, nalodeine, alvimopan, methylnatrexone, naloxegol, acamprosate, disulfiram, topiramate, gabapentin, methadone, buprenorphine, or lofexidine, or a GLP-1 agonist. The one or more processors can receive a request from the user indicating an onset of the behavior associated with the substance use and initiate responsive to receiving the request, the instructions for activities to address maladaptive behaviors associated with substance use in the user. The one or more processors can determine a time at which to provide the instruction to perform the activity to refrain from selecting one or more of the plurality of memory words and/or the plurality of substance use words where providing the instruction to perform the activity to refrain from selecting one or more of the plurality of substance use words and/or the plurality of memory words further comprises providing the instruction at the determined time. The one or more activities can be performed over a period of 1 minute, 2 minutes, 3 minutes, 5 minutes, 7 minutes, 10 minutes or longer. The substance use can include at least one of alcohol abuse disorder (AUD), opioid abuse disorder (OUD), or drug abuse. The prompt can be provided following at least 1 day, 5 days, 1 week, or one month following the providing of the instruction to perform an activity to provide information on a future event. The one or more processors can provide, prior to the instruction to perform an activity to refrain from selecting one or more of the plurality of memory words and one or more of the plurality of substance use words, an instruction to refrain from selecting the one or more of the plurality of substance use words.

Another aspect of the present disclosure is directed towards methods for treating or ameliorating a substance use disorder (SUD) in a user in need thereof. The methods include administering to the user a treatment including a digital therapeutic. In some aspects, administering the digital therapeutic includes identifying, for a user with a substance use disorder, a profile including a plurality of substance use words. Administering the digital therapeutic includes presenting a plurality of memory words not related to the substance use, and an instruction to memorize the plurality of memory words. In some aspects, administering the digital therapeutic includes providing an instruction to perform an activity to refrain from selecting one or more of the plurality of memory words and one or more of the plurality of substance use words.

In various implementations, administering the digital therapeutic includes providing an instruction to perform an activity to provide information on a future event. The future event can be related to the user's health, relationships, values, finances, enjoyment, charitable contributions, sobriety, or lifestyle. The future event can be at least one day, one week, one month, one year or longer in the future. Administering the digital therapeutic may include, prior to the instruction to perform the activity, providing an instruction for the user to select a category of the future event. The category of the future event can be selected from the user's health, relationships, values, finances, enjoyment, charitable contributions, sobriety, small steps, achievement, future gratitude, challenges, lifestyle, or overcoming maladaptive behavior associated with substance use. In various implementations, administering the digital therapeutic includes providing an instruction to select a timeframe for the future event. The future event may occur in less than a month. The future event may occur in more than a month. Administration of the digital therapeutic may include providing an instruction the user to generate one or more visual cues based on the information on the future event provided by the user. The visual cue can be an image or text, including free text provided by the user. Administering the digital therapeutic can include provide a prompt containing the visual cue related to the information on the future event and an instruction to perform an activity related to the visual cue. The prompt can be a timeline identifying more than one visual cues. The timeline can automatically update based on the time tagged to the visual cue. The activity can include an audio recording or a textual description.

Administering the digital therapeutic can include providing a subsequent instruction to perform an activity to refrain from selecting one or more of the plurality of memory words and/or one or more of the plurality of substance use words and providing a prompt including the one or more visual cues related to the information on the future event and an instruction to perform an activity related to the one or more visual cues. The prompt can be a timeline identifying more than one visual cues. The timeline can automatically update based on the time tagged to the visual cue. The activity can include an audio recording or a textual description.

In various implementations, administering the digital therapeutic can include providing a subsequent instruction to perform an activity to refrain from selecting one or more of the plurality of memory words and/or one or more of the plurality of substance use words. Administering the digital therapeutic can include providing, following performance of the activity to refrain from selecting one or more of the plurality of memory words and presentation of one or more of the plurality of substance use words, the prompt related to the information on the future event. Administering the digital therapeutic can include providing, prior to the instructions to perform an activity to refrain from selecting one or more of the plurality of memory words and one or more of the plurality of substance use words, an instruction to perform an activity to refrain from selecting one or more of the plurality of substance use words.

In various implementations, administering the digital therapeutic can include providing, an instruction to perform an activity to select all of the memory words from a plurality of words. Administering the digital therapeutic can include providing, an instruction to perform an activity to select one or more of the memory words from a plurality of words. Administering the digital therapeutic can include determining a score indicating a percentage of correct interactions with the memory words and then selecting, by the digital therapeutic, the plurality of memory words based on the score. In various implementations, Administering the digital therapeutic can include determining a score indicating correct interactions, speed of the user selections, or both during performance of the activity to select all memory words from a plurality of words. Administering the digital therapeutic can include determining, responsive to the score satisfying a threshold, an increase in a number of words for a subsequent activity to refrain from selecting a plurality of memory words, an activity to refrain from selecting a plurality of memory words, or an activity to select all memory words from a plurality of words. Administering the digital therapeutic can include displaying the score indicating at least one of a percentage of correct interactions, speed of user selections, or both during one or more activities.

Administering the digital therapeutic can include determining a score indicating correct interactions, speed of user selections, or both during performance of one or more activities and then selecting, by the one or more processors, a subsequent plurality of substance use words or a subsequent plurality of memory words, or both based on the score. The score can be determined by the number of correct interactions, speed of user selections, or both during performance of the activity to select all the memory words from a plurality of words. Administering the digital therapeutic can include determining a score indicating correct interactions, speed of the user selections, or both during performance of the activity to refrain from selecting the plurality of memory words and/or the plurality of substance use words. Administering the digital therapeutic can include determining, responsive to the score satisfying a threshold, an increase in a number of words for a subsequent activity to refrain from selecting the plurality of memory words and/or the plurality of substance use words.

Administering the digital therapeutic can include generating, one or more cue words or images based on the information on the future event provided by the user where the prompt related to the information on the future event comprises the one or more cue words or images.

In various implementations, administering the digital therapeutic can include providing an instruction to perform the activity to refrain from selecting one or more of the plurality of substance use words and/or the one or more of the plurality of memory words can further include providing, by the digital therapeutic, an instruction to perform an activity for the user to select one or more of a plurality of neutral words unrelated to substance use and different than the memory words. Administering the digital therapeutic can include providing the instruction to perform an activity to refrain from selecting one or more of the plurality of substance use words or memory words comprises presenting the one or more of the plurality of words for less than 1 second, less than 2 seconds, less than 3 seconds, 5 seconds, or equal to or greater than 10 seconds. The instruction to perform an activity can be provided to the user by the one or more processors at least once a day, at least once every 2 days, at least once every 5 days, or at least once a week. The one or more activities can be performed over a period of 1 minute, 2 minutes, 3 minutes, 5 minutes, 7 minutes, 10 minutes or longer. The prompt can be provided, by the one or more processors, at least 1 day, 5 days, 1 week, or one month following the providing of the instruction to perform an activity to provide information on a future event.

Administering the digital therapeutic can include receiving a request from the user indicating an onset of the behavior associated with the substance use and initiating, by the digital therapeutic, responsive to receiving the request, the instructions for activities to address maladaptive behaviors associated with substance use in the user. Administering the digital therapeutic can include determining a time at which to provide the instruction to perform the activity to refrain from selecting one or more of the plurality of memory words and/or the plurality of substance use words where providing the instruction, by the digital therapeutic, to perform the activity to refrain from selecting one or more of the plurality of substance use words and/or the plurality of memory words further comprises providing the instruction at the determined time.

In various implementations, the user can be administered an effective amount of a medication comprising at least one of naloxone, naltrexone, nalmefene, samidorphan, nalorpine, levallorphan, nalodeine, alvimopan, methylnatrexone, naloxegol, acamprosate, disulfiram, topiramate, gabapentin, methadone, buprenorphine, or lofexidine, or a glp-1 agonist. In various implementations, the substance use includes at least one of alcohol abuse disorder (AUD), opioid abuse disorder (OUD), or drug abuse.

In various implementations, the SUD in the user is ameliorated when the user experiences a decrease in Obsessive Compulsive Drug Use scale (OCDUS) metric following administration of the digital therapeutic. The SUD in the user can be ameliorated when the user experiences an improvement in a Drug Abstinence Self-Efficacy Scale (DASES) metric following administration of the digital therapeutic. The SUD in the user can be ameliorated when the user experiences an improvement in a modified Visual Analogue Scale (MVAS) rating for cravings following administration of the digital therapeutic. The SUD in the user can be ameliorated when the user experiences a decrease in self-reported substance use following administration of the digital therapeutic. The SUD in the user can be ameliorated when the user experiences an improvement in an Opioid Substitution Treatment Quality of Life (OSTQOL) scale following administration of the digital therapeutic. The user can be in treatment for substance use, at least in partial concurrence with the administration of the digital therapeutic. The user can be prescribed a medication for substance use. The substance use can be decreased in the user following administration of the digital therapeutic. The SUD in the user can be ameliorated when the user exhibits an improvement in at least one of a Recovery Empowerment Scale, Perceived Stress Scale, OSTQOL, MVAS, or self-reported total substance use with the timeline follow-back (TLFB) procedure following administration of the digital therapeutic. The digital therapeutic can be administered at least daily, every 2 days, or weekly. The digital therapeutic can be administered for at least one week, one month, or 3 months. Administering the digital therapeutic can include updating the profile of the user using the performance of the user in the activity.

### 1. Overview

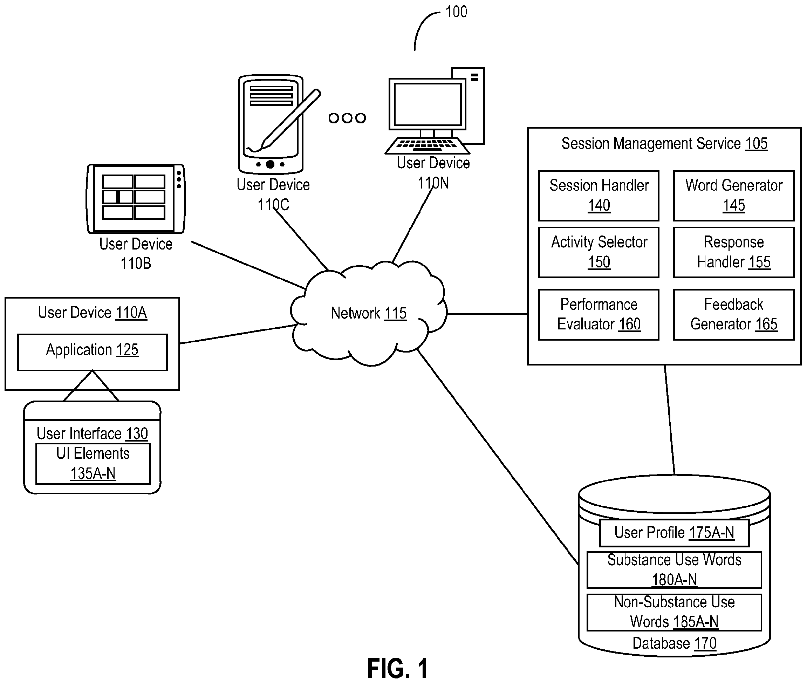

Referring now to FIG. 1, depicted is a block diagram of a system 100 for presenting interactive sessions to address maladaptive behaviors associated with substance use in users. In an overview, the system 100 may include at least one session management service 105 and a set of user devices 110A-N (hereinafter generally referred to as user devices 110), communicatively coupled with one another via at least one network 115. At least one user device 110 (e.g., the first user device 110A as depicted) may include at least one application 125. The application 125 may include or provide at least one user interface 130 with one or more user interface (UI) elements 135A-N (hereinafter generally referred to as UI elements 135). The session management service 105 may include at least session handler 140, word generator 145, activity selector 150, response handler 155, performance evaluator 160, and feedback generator 165, among others. The word generator 145 can include words provided in a database or words that are selected from a glossary of terms that are indication specific. In some aspects, the user may input relevant words that are related to the user's specific substance use. In other aspects, the user may rate the relevancy of words from a potential list of words provided by the word generator 145. In yet another aspect, eye-tracking or biometrics may be measured by the user device to determine which words are the most salient to the user for inclusion in a database to be selected by the word generator. In another aspect, the word generator 145 can generate new words in real time, possibly using generative AI machine learning model, without using pre-generated words or needing a database to store such words.

The session management service 105 may include or have access to at least one database 170. The database 170 may store, maintain, or otherwise include one or more user profiles 175 A-N (hereinafter generally referred to as user profiles 175). Collectively, the user device 110 and the session management service 105 may be part of a computing system to provide the application 125.

In further detail, the session management service 105 may (sometimes herein generally referred to as a service) be any computing device comprising one or more processors coupled with memory and software and capable of performing the various processes and activities described herein. The session management service 105 may be in communication with the one or more user devices 110 and the database 170 via the network 115. The session management service 105 may be situated, located, or otherwise associated with at least one server group. The server group may correspond to a data center, a branch office, or a site at which one or more servers corresponding to the session management service 105 is situated. The session management service 105 may be situated, located, or otherwise associated with one or more of the user devices 110. Some components of the session management service 105 may be located within the server group, and some may be located within the client device. For example, the session management service 105 may operate or be situated on the user device 110, and the activity selector 150 may operate or be situated on the server group.

Within the session management service 105, the session handler 140 may initiate sessions for a user by the application 125. The word generator 145 may generate words from the database 170 for instructions for the sessions. The activity selector 150 may select an activity for the user for the instructions. The response handler 155 may receive a response from the user in response to the instructions and the activity. The performance evaluator 160 may evaluate a performance of the user based on the response. The feedback generator 165 may provide the user with feedback via the application 125 based on results of the performance evaluator 160.

The user device 110 (sometimes herein referred to as an end user computing device or client device) may be any computing device comprising one or more processors coupled with memory and software and capable of performing the various processes and activities described herein. The user device 110 may be in communication with the session management service 105 and the database 170 via the network 115. The user device 110 may be a smartphone, other mobile phone, tablet computer, a wearable device (e.g., smart watch, eyeglasses), or laptop computer. The user device 110 may be used to access the application 125. In some embodiments, the application 125 may be downloaded and installed on the user device 110 (e.g., via a digital distribution platform). In some embodiments, the application 125 may be a web application with resources accessible via the network 115.

The application 125 executing on the user device 110 may be a digital therapeutics application and may provide the sessions (sometimes herein referred to as a therapy session) to address substance use disorders (SUDs). The user of the application 125 may be diagnosed with, or at risk of, a SUD. For example, the user may be using a substance more frequently and in larger amounts, developing health problems such as respiratory issues, or having withdrawal symptoms, among others. The causes of developing SUDs can include genetic, behavioral, environmental, physiological, and psychological factors, among others. For example, individuals with a family history of SUDs may have an increased susceptibility to substances and are at a higher risk of developing an addiction. In another example, socioeconomic status such as economic hardship and poverty may contribute to the usage of substances as a coping mechanism.

Examples of SUDs include opioid use disorders, alcohol use disorders, narcotic use disorders, among others. SUDs can lead to physical health, mental health, social and economic, and other issues. Effects on physical health may include, for example, heart disease (e.g., hypertension, arrhythmia, or cardiomyopathy), reduced lung function, lung damage, brain damage (e.g., neurotoxicity), or immunosuppression, among others. The condition may impede or hinder relationships, such as facing social stigma and may lead to economic losses such as unemployment.

The application 125 may be used to provide sessions to mitigate substance use in the user. The sessions may be presented to the user as a result of the user inputting information (e.g., substance use, lifestyle, work schedule). The sessions may include a variety of activities targeted to mitigating substance use such as inhibitory control training, working memory training, and thinking about future events. The sessions may include any number of activities in any order, and may be generated based on the information input by the user. By providing the user the digital therapeutics (e.g., the intervention) through the application 125, the adverse effects of SUDs can be addressed.

The user may be at least partially concurrently receiving a treatment to address the condition, or side effects of the condition, at least partially concurrently with the interventions provided by the application 125. For example, the user may be receiving treatment for the SUD. The user may be receiving a treatment at least partially concurrently with any number of sessions, or any combination thereof. The treatment can include a taking of a medication. The medication may be at least orally administered, intravenously administered, or topically applied. For example, the medication may include SUD treatment medications (e.g., naloxone, naltrexone, nalmefene, samidorphan, nalorpine, levallorphan, nalodeine, alvimopan, methylnatrexone, naloxegol, acamprosate, disulfiram, topiramate, gabapentin, methadone, buprenorphine, or lofexidine, or a GLP-1 agonist), among others. The application 125 may increase the efficacy of the medication that the user is taking to address the condition. The treatment can include cognitive behavioral therapy (CBT), contingency management, therapy, rehabilitation programs, or support groups, among others.

The application 125 can include, present, or otherwise provide a user interface 130 including the one or more UI elements 135 to a user of the user device 110 in accordance with a configuration on the application 125. The UI elements 135 may correspond to visual components of the user interface 130, such as a command button, a text box, a check box, a radio button, a menu item, and a slider, among others. In some embodiments, the application 125 may be a digital therapeutics application and may provide one or more sessions (sometimes referred to herein as a therapy session) via the user interface 130 for addressing substance use disorders in users.

The database 170 may store and maintain various resources and data associated with the session management service 105 and the application 125. The database 170 may include a database management system (DBMS) to arrange and organize the data maintained thereon. The database 170 may be in communication with the session management service 105 and the one or more user devices 110 via the network 115. While running various operations, the session management service 105 and the application 125 may access the database 170 to retrieve identified data therefrom. The session management service 105 and the application 125 may also write data onto the database 170 from running such operations.

Such operations may include the maintenance of the user profile 175 (sometimes herein referred to as a subject profile). The user profile 175 can include information pertaining to a condition of a user (e.g., SUD), as described herein. For example, the user profile 175 may include information related to the severity of the condition, occurrences of the condition (such as occurrences of symptoms associated with the condition), medications or treatments the user takes for the condition, and/or a duration of the condition, among others. The user profile 175 can be updated responsive to a schedule, periodically (e.g., daily, weekly), responsive to a change in user information (e.g., input by the user via the user interface 130 or learned from the user device 110), or responsive to a clinician (e.g., a doctor or nurse) addressing the user's condition, among others.

The user profile 175 can store and maintain information related to a user of the application 125 through user device 110. Each user profile 175 may be associated with or correspond to a respective user of the application 125. The user profile 175 may contain or store information for each session performed by the user. The information for a session may include various parameters, actions, images, prompts, or selections or actions of previous sessions performed by the user and may initially be null. The user profile 175 can enable streamlined communications to the user by presenting a session to the user which, based on at least the user profile 175, is most likely to aid the user in addressing the SUD in the user. This directed approach can reduce the need for multiple communications with the user, thereby reducing bandwidth and increasing the benefit of the user-computer interaction.

In some embodiments, the user profile 175 may identify or include information on a treatment regimen undertaken by the user, such as a type of treatment (e.g., therapy, pharmaceutical, or psychotherapy), duration (e.g., days, weeks, or years), and frequency (e.g., daily, weekly, quarterly, annually), among others. The user profile 175 may be stored and maintained in the database 170 using one or more files (e.g., extensible markup language (XML), comma-separated values (CSV) delimited text files, or a structured query language (SQL) file). The user profile 175 may be iteratively updated as the user provides responses, makes selections, and performs actions related to the session. The user profile 175 may also be updated based on words generated by the word generator 145 and activities performed by the user chosen by the activity selector 150. For example, the user profile 175 can be updated with responses received by the response handler 155.

The database 170 may store substance use words 180A-N (hereinafter generally referred to as substance use words 180). The substance use words 180 may be words associated with substance use. For example, the substance use words 180 can include needle, drug, or opioid among others. The user profile 175 may include substance use words 180 based on the information related to the user. The database 170 may also store memory words 185A-N (hereinafter generally referred to as memory words 185). The memory words 185 include words that are not related to substance use. For example, the memory words 185 can include dog, whale, ball, apple, or computer, among others. The database 170 may store neutral words.

### 2. Selection Activities

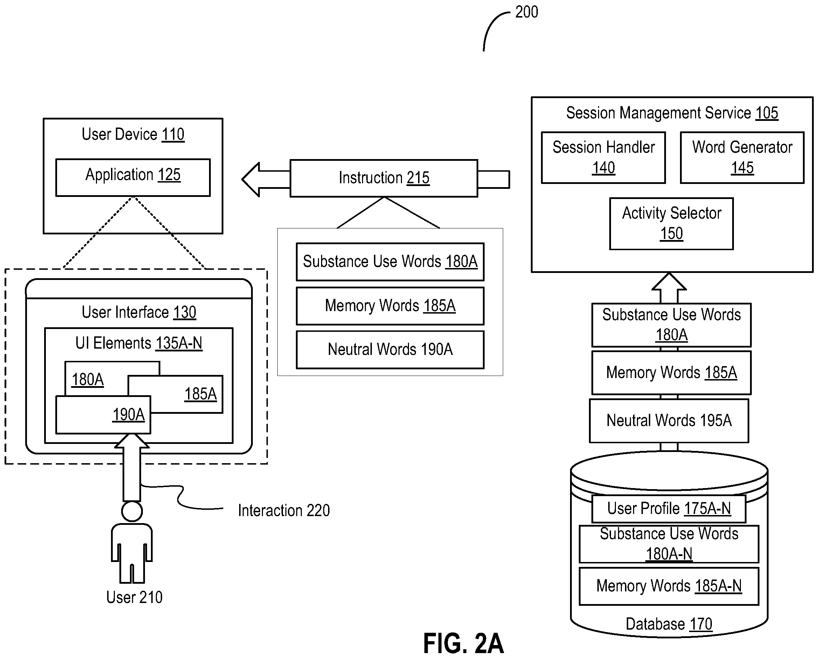

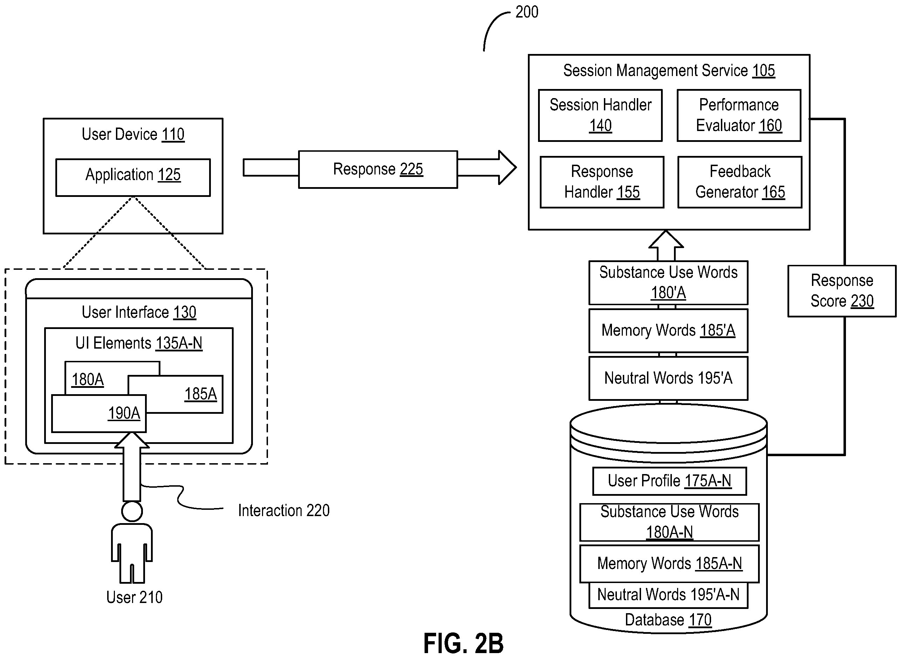

Referring now to FIGS. 2A-B, depicted are block diagrams for a process 200 to provide a session to a user 210 to perform an activity by the system 100. The process 200 may include or correspond to operations performed by the system 100 to receive and process data provided by the user 210. Starting with FIG. 2A, under the process 200, the word generator 145 can generate words from the database 170 to use in the activity chosen by the activity selector 150. The session combines a substance use word activity and a memory word activity into a single selection activity, meaning that a substance use word may be shown to the user or a memory word may be shown to the user. Combining substance use word and memory word activities can target overlapping neural network substrates, which functions to train two subdomains of executive control (e.g., inhibitory control and working memory) simultaneously, thus preparing the prefrontal cortex and limbic regions of the brain for plastic changes by simulating critical brain development time periods. An activity involving both substance use words and memory words may cause the user 210 to maintain focus and induce plasticity in the brain. For example, an instruction to the user 210 may include a message directing the user 210 to refrain from selecting both the memory words 185 and/or the substance use words 180 in the same activity but not necessarily on the same screen. In this case, one screen with a memory word may be presented to the user, and then a subsequent screen with a substance use word may be presented to the user, and the user would receive a correct score by not selecting with either screen. The instruction may also include directions to the user 210 to select one or more of a set of neutral words. The set of neutral words are unrelated to substance use and different than the memory words 185 used in the instruction. For example, the user 210 may withhold interacting (e.g. selecting) with the user interface 130 upon presentation of either the memory words 185 or the substance use words 180, but may select neutral words, in order to receive a correct score.

The selection activity trains the user 210 to strengthen inhibitory control and break up stimulus-action schemas such as triggers of the user 210 leading to drug use. The substance use word activity can break up associations of the user 210 with drugs, and allow new stimulus-action associations to be formed. The substance use word activity may also prepare the brain for plastic changes in the prefrontal cortex and limbic regions.

The substance use word activity may be selected by the activity selector 150 in combination with the memory word activity as a single activity. However, the activity selector 150 may select a substance use word activity as a single activity for the user to perform, meaning only substance use words and no memory words will be shown to the user. For example, this may occur to introduce the user to the treatment or to ease the user into a subsequent combined substance use word and memory word activity. Similarly, the activity selector 150 may select a memory word activity as a single activity for the user to perform, meaning only memory words will be shown to the user and no substance use words. Responsive to the activity selector 150 selecting the selection activity, the session handler 140 generates the instruction 215. The instruction 215 can include both substance use words 180 and memory words 185 generated by the word generator 145. The instruction 215 may include a message to perform a substance use word and memory word activity and refrain from selecting one or more of the substance use words 180 and/or memory words 190.

In some aspects, a single activity or session may include a plurality of rounds including a plurality of trials. Each trial may include the display of a single word. For example, a first trial may include displaying a substance use word, followed by a second trial displaying a memory word, followed by a third trial displaying a neutral word. However, in some embodiments, a single trial may include multiple words including multiple substance use words, multiple memory words, and/or multiple neutral words. The activity may include 1-20 rounds, 2-10 rounds, or 3-5 rounds per session. The activity may include 10-200 trials, 20-150 trials, 50-100 trials, or 70-80 trials per round. The number of trials per round or number of rounds per session depends on the amount of time given for a trial or round or session and the particular user, such as a period of 1 minute, 2 minutes, 3 minutes, 5 minutes, 7 minutes, 10 minutes or longer per round, as each user may vary in how much time they take to perform a trial, round, and/or session.

In order to generate the substance use words, the session handler 140 may first identify a SUD of the user based on the user profile 175. The user profile 175 can include a set of substance use words 180 based on the SUD of the user. Prior to generating the instruction 215, the word generator 145 may identify the words based on at least one of the user profile 175 or past sessions stored in the database 170. For example, the user profile 145 can contain conditions the user has had in the past and/or present which can be used to determine the intervention. Concurrently with generating the instruction 215, the user may be administered an effective amount of a medication to combat the SUD including at least one of naloxone, naltrexone, nalmefene, samidorphan, nalorpine, levallorphan, nalodeine, alvimopan, methylnatrexone, naloxegol, acamprosate, disulfiram, topiramate, gabapentin, methadone, buprenorphine, or lofexidine, or a glp-1 agonist. The word generator 145 may also generate words based on the medication the user is being administered.

In order to generate the memory words, the word generator 145 may select memory words 185 associated with the user profile 175. The instruction 215 may present the memory words 185 to the user 210. The memory words 185 may be unrelated to substance use. The instruction 215 may include a message to memorize the memory words 185.

In order to generate the neutral words, the word generator 145 may select neutral words based on the user profile 175, words that are unrelated to the substance use, and/or words that are different than the memory words 185, among other factors.

### 3. Visualization Activities

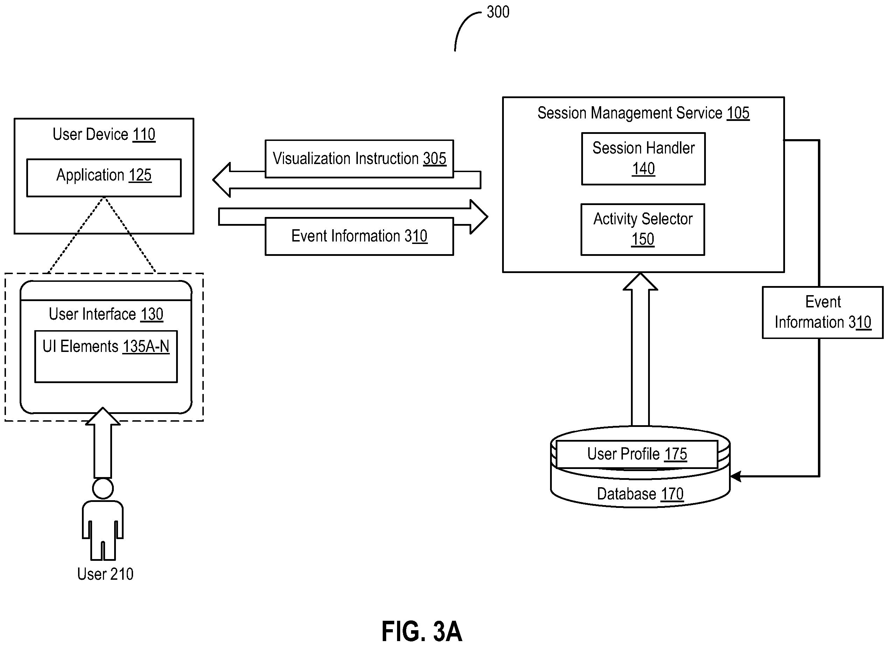

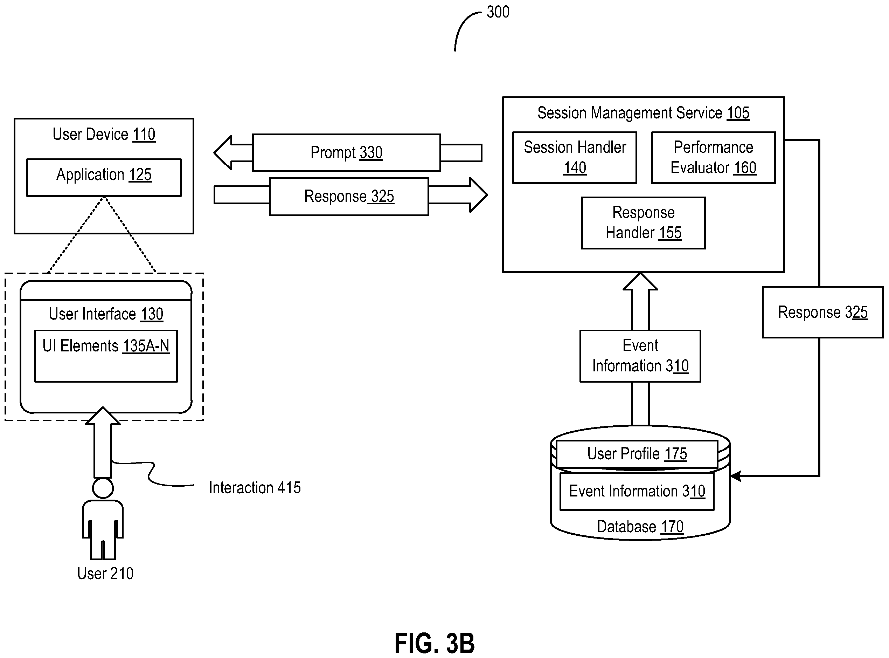

Referring now to FIGS. 3A and 3B, depicted are block diagrams for a process 300 to provide a session to a user 210 to perform a visualization activity by the system 100. Following the completion of a selection activity as described above, the process 300 may provide a session for the user to perform the visualization activity. The process 300 may include or correspond to operations performed by the system 100 to receive and process data provided by the user 210. Starting with FIG. 3A, under the process 300, the activity selector 150 may select the visualization activity and the session handler 140 may generate a visualization instruction 505 for the user 210 to perform the visualization activity. The visualization activity may provide goal-related context to the user 210 to capitalize on improvements in executive control function in the user 210 from the selection activity. The visualization activity may translate improvements from the selection activities to real-world contexts and can facilitate maintaining and updating associations the user 210 has with, for example, the substance use words 185.

Responsive to the activity selector 150 selecting the visualization activity, the session handler 140 may generate the visualization instruction 305. The visualization instruction 305 may instruct the user 210 to perform the visualization activity involving asking the user to provide information 310 on a future event. The visualization instruction 305 may be a text or audio description of the activity. The visualization instruction 305 may be generated, sent, and received in a same way as the other instructions described herein. The future event may be related to health, relationships, values, finances, enjoyment, charitable contributions, sobriety, small steps, achievements, future gratitude, challenges, lifestyle, or overcoming maladaptive behavior associated with the substance use of the user 210. The future event may be generally positive, such as birthdays, celebrations, weddings, graduations, goals, or objectives, among others. The future event may be at least one day, one week, one month, one year or longer in the future. The future event may be less than 1 week, 1 day, 12 hours, or one hour in the future. The visualization instruction 305 may present the user 210 with UI elements 135 to input the event information 310, such as when the future event might be occurring. For example, the user 210 may input an upcoming future event to be graduation and a date of the graduation. The event information 310 may then be stored in the database 170.

In some embodiments, prior to the session handler 140 generating the visualization instruction 305, the session handler 140 can provide an instruction to the user 210 to select a type of future event selected from at least one of the user's health, relationships, values, finances, enjoyment, charitable contributions, sobriety, small steps, achievement, future gratitude, challenges, lifestyle, or overcoming maladaptive behavior associated with substance use. In some embodiments, the session handler 140 can provide an instruction to the user 210 to select a category for the future event. The category can be selected from at least one of the user's health, relationships, values, finances, enjoyment, charitable contributions, sobriety, small steps, achievement, future gratitude, challenges, lifestyle, or overcoming maladaptive behavior associated with substance use. The future event can be tagged by category. In some embodiments, the session handler 140 provides the instruction to perform the visualization activity based on the category after the user 210 has performed a certain number of visualization activities. In various implementations, the visualization instruction 405 can include a request to select a timeframe for the future event. The future event may occur in less than one month or more than one month. The future event can be tagged by the timeframe. In some embodiments, the session handler 140 provides the instruction to perform the visualization activity based on the timeframe after the user 210 has performed a certain number of visualization activities.

Following performance of the visualization activity, the session handler 140 may generate a prompt 330 related to the event information 310. The prompt 330 may be used in subsequent sessions for the user to visualize the future event from a prior session. The prompt 330 may encourage the user 210 and remind the user 210 of cognitive gains made via the system 100. The prompt 330 may be provided by the session handler 140 to the user following at least 1 day, 5 days, 1 week, or one month following providing the visualization instruction 305 to the user 210, particularly after the user has performed a subsequent session(s) of the selection activities.

The prompt 330 may include one or more cue words or images based on the event information 310 provided by the user, to remind the user 210 of an upcoming future event. For example, responsive to the event information 310 including information on graduation, the cue may include the word "graduation" or "cap and gown" or an image of a graduation cap and gown. The user 210 may also input one or more self-generated cue words or images via the UI elements 130 and the cue words and images may be recorded. In some embodiments, the session handler 140 may instruct the user 210 to generate one or more visual cues based on the information on the future event provided by the user. In some embodiments, the session handler 140 may provide the user 210 with one or more visual cues to select from, and store the visual cues the user 210 selects.

The prompt 330 can be a timeline identifying more than one visual cue. For example, the prompt 330 can include an image of a birthday cake with the words "in one month" representing a family member's birthday, and an image of a plane with the words "in two months" representing a trip to visit a friend. The timeline can automatically update based on a time tagged to the visual cue. For example, the session handler 140 can request the user 210 to tag selected visual cues with a time, and generate the timeline based on the visual cues and the current time.

In some embodiments, the session handler 140 may send a request to the user to input information to generate the prompt 330. For example, the session handler 140 may request the user to provide textual reminders (e.g., cue words), a date and/or a timeframe of the future event, and a category of the future event and/or the prompt 330. For example, the prompt 330 can be categorized as at least one of the user's health, relationships, values, finances, enjoyment, charitable contributions, sobriety, small steps, achievement, future gratitude, challenges, lifestyle, or overcoming maladaptive behavior associated with substance use.

In some embodiments, the session handler 140 stores the prompt 330 following generation of the prompt 330. The session handler 140 can store the prompt 330 following each visualization activity. A collection of the prompts 330 may be displayed on the user device 110 for the user 210 to visualize various future events all at once. For example, the collection of the prompts 330 may be displayed as a future vision board on the user device 110.

In further reference to FIGS. 3A-3B, the prompt 330 may be provided to the user following the process 200, and/or the process 300. For example, the session handler 140 may provide an instruction to the user 210 to perform an activity to refrain from selecting one or more of the memory words 185 and/or one or more of the substance use words 180. Following performance of the selection activity, the session handler 140 may send the prompt 330 to the user device 110 for presentation to the user 210. The prompt 330 can include the one or more visual cues. The one or more visual cues may be a description of the future event or an image depiction of the future event. The cues can be in other formats such as audio. The prompt 330 may reinforce brain plasticity caused by the activity and reinforce changes within the executive control system of the brain.

Following the prompt 330, the session handler 140 may generate a subsequent activity for the user 210 to perform. The subsequent activity can be related to the visual cues. The subsequent activity can include an audio/video recording or a textual description. The audio/video recording or the textual description can be related to the visual cues. The application 125 may record an interaction 315 of the user 210 with the activity.

In some embodiments, the prompt 330 is provided following the performance of the selection activity. In some embodiments, the prompt 330 is provided prior to performing the selection activities. In some embodiments, the prompt 330 is provided during performance of the selection activities. As another example, the user 210 may be first provided the visualization activity, provided the prompt 330, and then instructed to perform a selection activity. In some aspects, the user may be instructed to perform a selection activity, followed by an instruction to perform a visualization activity, followed by a subsequent instruction to perform a selection activity, followed by the prompt 330.

### 4. Repeating Cycles of Selection Activities and Visualization Activities

For maximum benefit, the session handler 140 may initiate a selection activity, then a visualization activity with a storing of the prompt, then a subsequent selection activity and then a subsequent visualization activity including showing the prompt to the user, and this cycle may repeat multiple times over 1-24 hours, 1-7 days, 1-4 weeks, 1-12 months, or greater than a year.

In other embodiments, performance of the three activities may be performed in a variety of orders, a variety of times, and in multiple combinations based on the user profile 175. Each of the activities may be performed for a number of frequencies and/or a number of times (in second, minutes, etc.).

For example, in a given week, the user 210 may be provided with a substance use word and memory word instruction on a certain number of the days in the week to perform the combined selection activity. The activity may be performed for a certain number of times depending on the user profile 175. The visualization instruction 305 may be provided to the user 210 on a certain number of the days the substance use word and memory word instruction is also delivered. The visualization activity may be performed for a certain number of times based on the user profile 175. On the days the user 210 is provided with the substance use word and memory word instruction, the prompt 330 may be provided to the user 210 before, during, or after performance of the substance use word and memory word activity.

To implement this cycle, the activity selector 150 may select a selection activity or a visualization activity for the user 210 to perform. The activity selector 150 may also select a length of time the activity is performed over such as 1 minute, 2 minutes, 3 minutes, 5 minutes, 7 minutes, 10 minutes or longer. The time in which the session handler 140 sends a session may be at least once a day, at least once every 2 days, at least once every 5 days, or at least once a week.

The sessions generated by the session management service 105 described herein can reopen a window of plasticity in adults, typically limited to childhood. For example, the activity selector 150 alternating between substance use word and memory word activities can reopen the window. The session handler 140 can mimic key principles of learning observed during time periods in which an experience shapes a trait and behaviors are developed (e.g., plasticity) by providing sessions with various activities and words. For example, substance use word activities with prepotent responses, short response times, and indication-specific words as dictated by the session handler 140 and/or the feedback generator 165 can create a challenging and salient learning environment for the user to develop rapid adaptations under the conditions and focused attention.

### 5. Memory quiz

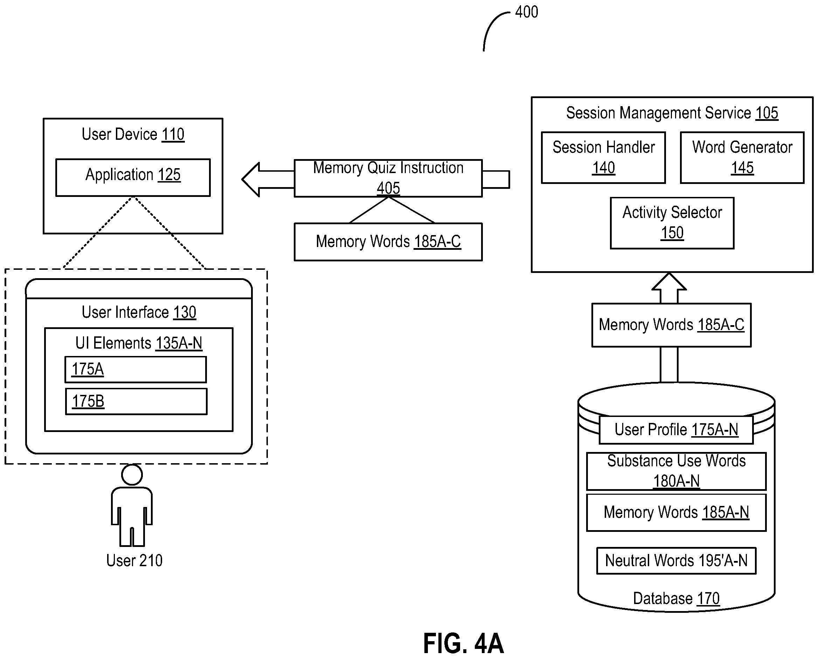

Referring now to FIGS. 4A-B, depicted are block diagrams for a process 400 to provide a session to a user 210 to perform a memory quiz activity by the system 100, which is provided following the selection activity. The process 400 may include or correspond to operations performed by the system 100 to receive and process data provided by the user 210. Starting with FIG. 4A, under the process 400, the activity selector 150 may select memory quiz and the session handler 140 may create, write, or otherwise generate a memory quiz instruction 405 based on the words. The memory quiz instruction 405 directing the user 210 to perform a memory quiz activity may reinforce learnings from the selection activity. For example, the selection activity may include a subset of the memory words 185 while the memory quiz activity includes all of the memory words 185.

The session handler 130 may generate the memory quiz instruction 405 responsive to the activity selector 150 selecting the memory quiz activity for the user 210 to perform. The memory quiz instruction 405 may include a message to perform the memory quiz activity by interacting with (e.g., select) all of the memory words 185 from a set of words. The set of words may include both memory words 185 and neutral words. The set of neutral words are different than the memory words 185 or the substance use words 180 and are unrelated to substance use. For example, the memory words 185 may be presented alongside the neutral words, and the memory quiz instruction 405 may instruct the user 210 to select only the memory words 185. In some embodiments, the memory quiz instruction 405 may instruct the user 210 to select one or more of the memory words 185. For example, the memory quiz activity may not include all of the memory words 185. The memory quiz instruction 405 may also include a subset of the memory words 185 for the user 210 to select. The memory quiz instruction 405 may be generated, sent, and received in a same way as the other instructions described herein.

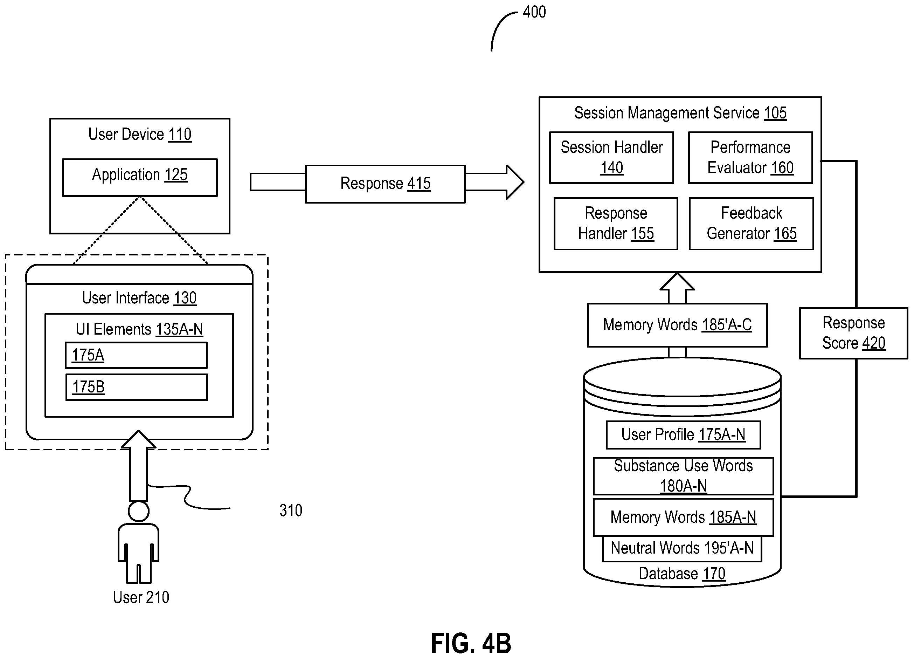

Moving onto FIG. 4B, upon receipt of the memory quiz instruction 405, the application 125 on the user device 110 can display, render, or otherwise present the memory quiz instruction 405 via the user interface 130. The application 125 may then prompt or direct the user 210 to provide a response 415 to the memory quiz instruction 405, such as performing the memory quiz activity. The response 415 may be provided by the user 210 performing an interaction 410 with the UI elements 135 on the user interface 130 showing the memory words 185 and the neutral words. Responsive to the memory word 185 appearing on the screen, the user 210 may select the memory words 185 which may be included in the interaction 410. The interaction 410 may be recorded by the application 125 and sent as the response 415. The interaction 410 may also record interactions of the user 210 with the neutral words. The interaction 410 may be recorded, received, and determined in a same way as other interactions described herein.

Responsive to the response score 420 satisfying a threshold, the feedback generator 165 may increase a number of words for a subsequent activity. The feedback generator 165 may instruct the word generator 135 to generate more memory words 185' for the subsequent activity. The memory words 185' may include the memory words 185, or may be different than the memory words 185 used in a previous session.

### 6. Examples

In one example, the user interfaces may be presented through the application. The user interface may be an indication of which activity the user is instructed to perform. The user interface may provide an instruction to the user regarding the activity. The user interface provides memory words for the user to memorize and refrain from selecting.

The user interface may provide an instruction for the user to memorize the memory words and refrain from selecting the memory words during a selection activity. The memory words may be provided on a screen of the user interface with an instruction to refrain selecting the memory words. The user interface may provide an instruction to refrain from selecting the substance use words. The user interface may provide an instruction to refrain from selecting both the substance use words and the memory words. The user interface may present one of the substance use words or the memory words in one region of the user interface. Following an interaction of the user with the substance use word or memory word, the user interface may remove the substance use word or memory word then present a subsequent memory word or substance use word in a different region of the screen than the prior memory word or substance use word. In some aspects, a substance use word is presented prior to the presentation of the memory word. In some aspects, a memory word is presented prior to the presentation of a substance use word. In some aspects, a neutral word that is unrelated to substance use is presented to the user interface in the same or different region than a prior substance use or memory word. The substance use word, memory word, or neutral word may be presented for a period of time on the user interface then removed before the subsequent word is presented. Alternatively, the substance use words, memory words, and/or neutral words may be presented on the user interface on a single screen. The period of time for the presentation of the memory words, substance use words, or neutral words may be less than 1 second, less than 2 seconds, less than 3 seconds, 5 seconds, or equal to or greater than 10 seconds. The user interface may indicate to the user that the activity is complete. The user interface may display a score to the user based on a performance of the user during the activity.

In one example, the user interfaces for a memory quiz activity may present a set of words including memory words for the user to select. The user interface may present the memory words alongside a set of neutral words with an instruction for the user to select the memory words. In some aspects, the user interface may include an instruction to refrain from selecting the neutral words. The memory words may be displayed for a period of time. The memory words and neutral words may be presented together on a single screen. The period of time for the presentation of the memory words may be less than 1 second, less than 2 seconds, less than 3 seconds, 5 seconds, or equal to or greater than 10 seconds. The user interface can show results to the user based on the interactions and a score based on the results of the activity.

In some examples, a user interface indicating scoring for the activities may be presented to the user following performance of, for example, the memory quiz activity, and/or the substance use word and memory word activities. The user interface may present an explanation to the user what a score is based on, including the percent, number, and/or timing of correct interactions and/or selections. The user interface may display the score to the user as well as metrics related to the score such as accuracy, timing of selections, number and/or percent of correct interactions and/or selections. The user interface may also indicate the challenge or difficulty of the activities and how they may affect the user's score. In some embodiments, the user interface provides for areas of improvement for the user, including activities where the user can improve performance. The user interface may indicate the words of the incorrect interactions and/or selections.

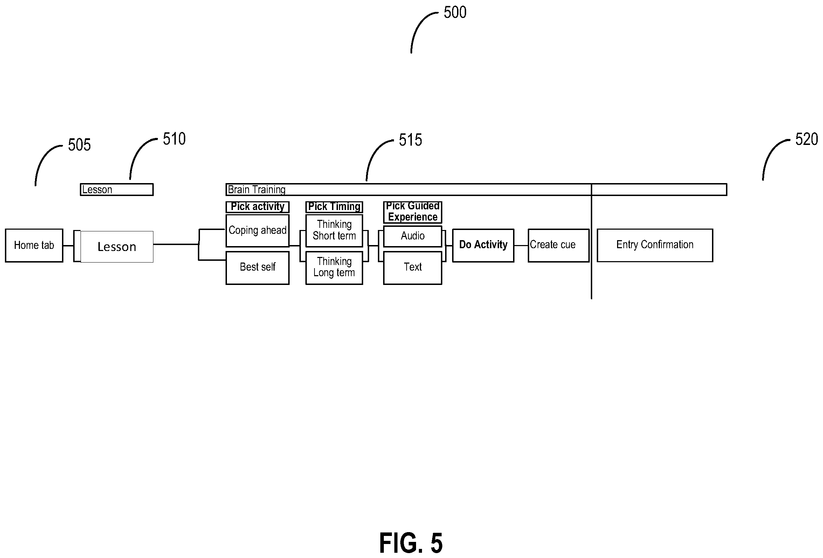

FIG. 5 depicts a flow diagram for a visualization activity, in accordance with an illustrative embodiment. The user interfaces in the set 500 may be part of the application, and presented through the user interface 130. The user interface 505 may be a home page (e.g., start page) for the user to begin the visualization activity. The user interface 510 may introduce the visualization activity and any subsequent activities (e.g., selection activity). The user interface 515 may display the visualization activity for the user to perform. The user interface 520 may be an ending screen of the visualization activity and/or the subsequent activities.

In some examples, the user interface may present a visualization activity and indicate to the user which activity the user is instructed to perform. The user interface may present instructions for engaging in a visualization activity to the user. The user interface may provide instructions for the user to imagine a future event. The user interface may provide instructions for the user to input information on a future event. The user interface may present the user with specific questions regarding the future event, such as the timing of the event, the location of the event, who is present at the event, or how the user feels upon accomplishing the event, among others. The user interface may present the user with a question or request for the user to input information regarding the event, such as a description or image of the event.

In another example of a user interface for a visualization activity, the user interface can provide the user with options for a timeframe (e.g., one day, one week, one month, or more than one year, etc.) of the future event of the visualization activity. The user interface can present the user with the option of selecting a topic or category (i.e.: health, relationships, values, finances, enjoyment, charitable contributions, sobriety, small steps, achievement, future gratitude, challenges, lifestyle) for the future event and instructions to visualize an event related to the category. In some aspects, the user interface may present the user with an instruction to select an upcoming challenge in the user's life and how the user intends to handle the future event or challenge. The user interface may present an option for selecting the time frame of the future event or challenge. For example, the user interface may present options of specific timeframes (e.g., one day, one week, one month, or more than one year, etc.) for the future event or challenge.

In one example, the user interface can include inputs for the user to create a prompt for the visualization activity that may include a reminder or image related to the future event and the date of the future event. The user interface may request the user to select a category (i.e.: health, relationships, values, finances, enjoyment, charitable contributions, sobriety, small steps, achievement, future gratitude, challenges, lifestyle). The user interface may include an instruction for the user to enter information relating to the future event, such as a description or image of the event.

In some examples, the user interface can include inputs for the user to create a cue and/or reminder related to the future event. The cue may be text, image or audio-based content. The user interface may include an input or instruction for the user to provide a reminder for the event and a description or image related to the event. The user interface may request the user to input a timing of the future event.

Upon the user providing the input related to the cue, the user interface may indicate to the user that a new cue has been created. The user interface may display a future board and inform the user that the cue has been added to the user's future board. The cue may be provided on the user's future board on a timeline indicating the date or time of the event (e.g., one day, one week, one month, or more than one year, etc.). The user interface may be a screen indicating the completion of the visualization activity and/or a starting screen for a subsequent activity (e.g., substance use word activity).

In one example, the user interface may present the future board following the user performing one visualization activity. The user interface may present the future board with multiple cues input by the user. The user interface may present the future board after the user has performed additional selection activities and/or visualization activities. The future board can be sorted by the timeframe (e.g., one day, one week, one month, or more than one year, etc.) that the future event is occurring and/or the category of the cue (i.e.: health, relationships, values, finances, enjoyment, charitable contributions, sobriety, small steps, achievement, future gratitude, challenges, lifestyle). The cues may be provided along the timeline and presented in chronological order or within a grouping of cues that will occur within a similar time frame (e.g., one day, one week, one month, or more than one year, etc.). The cues related to the visualization activities may be removed from the future board or moved to a separate position or grouping upon completion or passage of the future event. The user interface may include an instruction to interact with one or more cues displayed on the future board, including an instruction to indicate that the future event has been completed or has passed. The cues may be text, audio, or visual based content related to the future event.

### 9. Process

Referring to FIGS. 1-4B, the instruction 215 may take various formats associated with the application 125. In some embodiments, the instruction 215 may be displayed, rendered, or otherwise presented via the user interface 130 of the application 125 to the user 210. In some embodiments, the instruction 215 may include a short message service (SMS) (e.g., text message) or multimedia message service (MMS) (e.g., audio message, video message). For example, the instruction 215 may include a link to open the application 125 directing the user to perform the activity.

The interaction 220 can include actions such as a physical manipulation of the user device 110, actuation of the UI elements 135, or viewing of the substance use word 180 or memory word 185 or neutral word 190, among others. The interaction 220 can include an action of moving, tapping, sliding, nodding, shaking, tilting, or orienting the user device 110 towards a side of the user device 110. A side of the user device 110 can refer to a boundary created by the user interface 130 associated with the user device 110, such as a side of a rectangular screen.

The interaction 220 can include an action such as touching, pressing, or otherwise actuating a UI element 135 of the user interface 130 associated with at least one of the substance use word 180, memory word 185, or neutral word 190. For example, the user 210 can provide one or more interactions 220 through the application 125 running on the user device 110 by actuating one or more of the UI elements 135 as described herein. The user 210 can provide the interaction 220 by pressing a button associated with the application 125 and displayed via the user interface 130. In some embodiments, one or more first UI elements 135A can be associated with the substance use words 180 and one or more second UI elements 135B can be associated with the memory words 185 and one or more third UI elements 190 can be associated with the neutral words 190. In some embodiments, the interaction 220 can include the user refraining from selecting or actuating the one or more UI elements 135 during a selection activity for a period of time while the substance use word 180, memory word 185, or neutral word 190 are presented by the user interface 135. The period of time in which the words are presented and the user refrains from selecting the words may be 1 second, less than 2 second, less than 3 seconds, 5 seconds, or equal to or greater than 10 seconds.

The application 125 may monitor for at least one interaction 220 with the substance use words 180, the memory words 185, and/or the neutral words 190. The application 125 can monitor during the session responsive to presentation of the substance use words 180, the memory words 185, and/or the neutral words 190, or responsive to receiving the interaction 220. The application 125 can monitor for receipt of the interaction 220. The application 125 can monitor for the interaction 220 through the user interface 130 or through sensors associated with the user device 110, among others. The application 125 can receive multiple interactions 220 during a session. For example, the application 125 can monitor for a series of interactions 220 provided by the user 210 during the session. The application 125 may monitor and record information related to the received interactions 220. For example, the application 125 may monitor and record a time of an interaction 220, a duration of an interaction 220, a sequence of interactions 220, the substance use words 180 and/or the memory words 185 and/or neutral words 190 associated with the interaction 220, and/or the delay time between the presentation of the substance use words 180, the memory words 185, the neutral words 190, and the selection of those words, among others.

Upon the user 210 providing the interaction 220, the application 125 may generate at least one response 225. The response 225 can identify the interaction 220. The response 225 can include the information about the interaction 220, such as a duration of the interaction 220, a time of the interaction 220, the substance use words 180 and/or memory words 185 and/or neutral words 190 associated with the interaction 220, and/or a delay time between the presentation of the substance use words 180 and/or memory words 185 and/or neutral words 190 and the selection of those words, among others. The application 125 can generate the response 225 for transmittal to the session management service 105. The response 225 can be in a format readable by the session management service 105, such as an electronic file readable by the session management service or data packets readable by the session management service 105, among others.

The response handler 155 can receive, identify, or otherwise detect a response 225 identifying the interaction 220. The response handler 115 can receive the response 225 from the application 125. The response handler 155 can receive the response 225 at scheduled time intervals or as the interactions 220 occur during the session. The response handler 155 can query or ping the application 125 for the response 225. The response handler 155 can receive multiple responses 225 during a time period. For example, the response handler 140 can receive a first response 225 indicating a first interaction 220 and a second response 225 indicating a second interaction 220.

The response handler 155 can store the response 225 including the interaction 220 in the database 170. The response handler 155 can store information related to the response 225, including a time of the response 225, actions associated with the interaction 220, the user profile 175 associated with the response 225, and the substance use words 180 and/or memory words 185 and/or neutral words 190 associated with the response 225, among others. The response 225 may include or identify the interaction 220 by the user 210 with the substance use words 180 and/or memory words 185 and/or neutral words 190. The response 225 may include a time for activity completion or a number of activities for activity completion. For example, the response 225 may include that the user completed a certain number of activities or spent a certain number of minutes to perform the action associated with the presentation of the substance use words 180 and/or memory words 185 and/or neutral words 190. The response 225 can include a total number of trial or rounds for completion of the session, a total time for completion of the session, and may also include a time of initiation of the session and a time of completion of the session. The response 225 may include the UI elements 135 interacted with during the duration of the presentation of the substance use words 180 and/or the memory words 185 and/or the neutral words 190. For example, the response 225 may include a listing of buttons, toggles, or other UI elements 135 selected by the user 210 at specified times during the presentation of the substance use words 180 and/or memory words 185 and/or neutral words 190. The response 225 may include other information, such as a location of the user 210 while performing the session, such as a geolocation, IP address, GPS location, or triangulation by cellular towers, among others. The response 225 may include measurements such as measurements of time, location, or user data, among others.

The performance evaluator 160 can calculate, generate, or otherwise determine the response score 230 associated with the response 225 based on the interaction 220 with the substance use words 180 and/or memory words 185 and/or neutral words 190. The response score 230 can indicate a level of correctness or conversely a level of error associated with the response 225. A high response score 230 can correlate with a high level of correctness in selecting the neutral words 190 and/or refraining from selecting the substance use words 180 and/or the memory words 185. A low response score 230 can correlate with a low level of correctness (e.g., high level of error). For example, a low response score 230 can indicate that the user 210 has selected substance use words 180 and/or memory words 185.

In determining the response score 230, the performance evaluator 160 may evaluate the response 225 based on the interaction 220. The performance evaluator 160 can determine the response score 230 based on a percentage of correct interactions with the memory words 185 and/or the substance use words 180 and/or the neutral words 190. The percentage of correct interactions may be based on a number, or rate, among others, that the user 210 interacted with the memory words 185 and/or the substance use words 180 and/or the neutral words 190 based on the interaction 220. For example, the performance evaluator 160 may calculate the response score 230 based on a number of interactions with the substance use words 180, a number of times a selection was withheld with the substance use words 180, a number of interactions with the memory words 185, a number of times a selection was withheld with the memory words 185, a number of interactions with the neutral words 190, and/or a number of times a selection was withheld with the neutral words 190.

In some embodiments, the response score 230 is determined based on correct interactions, speed of user selections, or both during the interaction 220. The correct interactions indicates a number, rate, or percentage, among others, of the user 210 correctly interacting with the neutral words 190 and refraining from selecting the substance use words 180 and memory words 185. The application 125 can record a speed between interactions which can be used to calculate the response score 230. For example, the time between the memory word 185 appearing on the user interface 130 and the user 210 incorrectly interacting with the memory word 185 can be used to calculate the response score 230. The response score 230 can also be displayed on the user interface 130 indicating at least one of a percentage of correct interactions, speed of user selections, or both during the interaction 220.

The feedback generator 165 may also produce, output, or otherwise generate feedback for the user 210 to receive via the application 125 operating on the user interface 130. The feedback generator 165 may generate feedback based on at least the response score 230, the user profile 175, the response 225, or the historic substance use word 180 and memory word 185 and neutral word 190 presentation. The feedback may include text, video, or audio to present to the user 210 via the application 125 displaying through the user interface 130. The feedback may include a presentation of the response score 230. The feedback may display a message, such as a motivating message, suggestions to improve performance, a congratulatory message, a consoling message, among others. In some embodiments, the feedback generator 165 may generate the feedback during the session being performed by the user 210. The feedback may include the response score 230 for display on the user interface 1430 including at least one of a percentage of correct interactions, speed of user selections, or both during the interaction 220.

Based on the response score 230, the feedback generator 165 can generate and select the subsequent substance use words 180' and memory words 185' and neutral words 190 to use in subsequent sessions. The subsequent substance use words 180' and memory words 185' and neutral words 190 can be associated with the user profile 175 and used in a subsequent activity. The user profile 175 can also be updated based on the response score 230 by the feedback generator 165. The interaction 220 may be recorded in the user profile 175, and subsequent activities and words may be generated by the session handler 140 based on at least one of the response score 230 and the interaction 220. Updates to the user profile 175 may reflect a progress and performance of the user 210, and may indicate to the session management service 105 to increase a difficulty of subsequent instructions or decrease a difficulty of subsequent instructions.

In some embodiments, the feedback generator 165 may also determine, based on the response score 230, whether to increase a number of substance use words and/or memory words and/or neutral words (e.g., increase a difficulty) of a subsequent activity of the session management service 105. For example, responsive to the response score 230 satisfying a threshold, the feedback generator 165 may update the user profile 175 to include the response score 230 responsive to receiving the response score 230 from the performance evaluator 160. The feedback generator 165 may determine whether the response score 230 satisfies the threshold. The user profile 175 may then indicate to the session handler 140 to increase the number of memory words in the subsequent session. The feedback generator 165 may also display the response score 230 on the user device 110.

Based on the response score 230, the feedback generator 165 may direct the session handler 140 to generate an instruction for the subsequent session to perform the activity to refrain from selecting memory words 185' and/or to refrain from selecting substance use words 180'. The memory words 185' may include the memory words 185, or may be different than the memory words 185 used in previous sessions. Words generated by the word generator 145 for subsequent sessions may be dictated by response scores of the first session, second session, and so on, particularly the response scores of the memory quiz. The feedback generator 165 may also update the user profile 175 based on the response score 230. The feedback generator 165 may store the response score 230 in the user profile 175, which can then be used for subsequent sessions initiated by the session management service 105. The feedback generator 165 may also increase a number of words for the user 210 to memorize in the subsequent session. For example, an amount of words of the memory words 185' may be greater than an amount of words of the memory words 185 to enhance a difficulty of the memory word activity for subsequent sessions. The feedback generator 165 may also decrease a number of words for the user 210 to memorize in the subsequent sessions to decrease a difficulty of the activity for subsequent sessions.

The progressive difficulty element determined by the performance evaluator 160 and the feedback generator 165 of the memory word training mirrors how skills are naturally acquired during developmental sensitive periods (e.g., plasticity periods in childhood). For example, the word generator 145 can increase a number of words generated responsive to the score generated by the performance evaluator 160. The visualization activities selected by the activity selector 150 and generated by the session handler 140 can leverage neural plasticity which underpins the formation of episodic memories throughout a lifespan of the user. The session handler 140 combining visualization with both substance use word and memory word supports neural plasticity across associated brain regions and can enhance a clinical impact of the digital therapeutic application. For example, the session handler 140 can generate sessions as dictated by the activity selector 150. The session handler 140 may then provide sessions chosen by the activity selector with words generated by the word generator 145 to support neural plasticity.

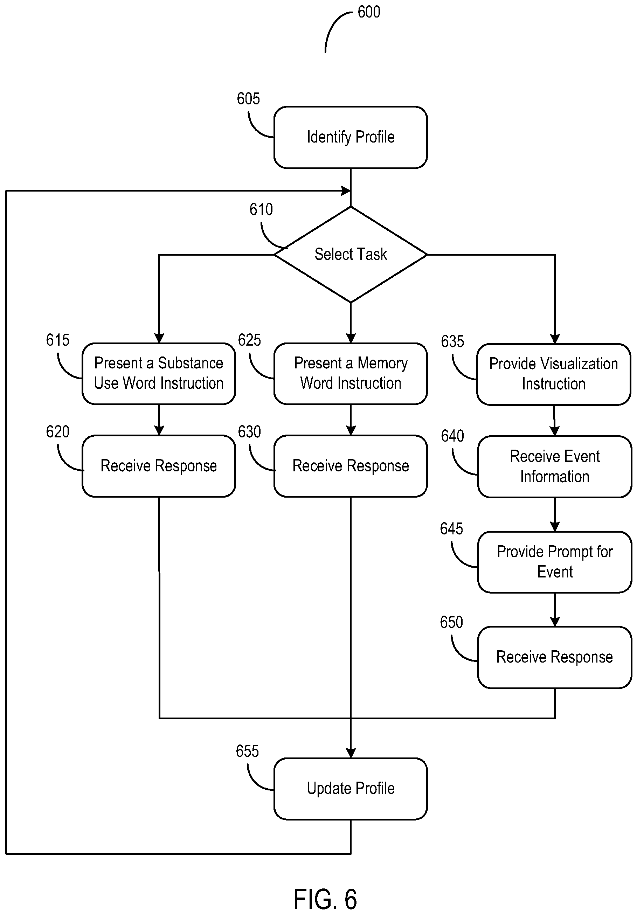

FIG. 6 depicts a flow diagram of a method 600 for providing activities to users to address substance use disorders in accordance with an illustrative embodiment. The method 600 may be performed by any components of the system 100, such as the session management service 105, the user device 110, or the user 210, among others. Under the method 600, one or more processors can identify a profile (605). The profile may be of the user 210 and may identify a specific substance use disorder and previous treatments of the user 210. The one or more processors can select an activity based on the profile (610). The activities may be based on the substance use disorder of the user 210. The one or more processors may present a substance use word instruction to the user 210 (615). The substance use word instruction may include substance use words and an instruction to withhold from selecting the substance use words. The one or more processors may receive a response to the substance use word instruction (620). The response may include interactions of the user 210 with the substance use words, memory words and/or neutral words. The one or more processors may present a memory word instruction (625). The memory word instruction may request the user 210 to interact with memory words presented to the user 210. The one or more processors can then receive a response to the memory word instruction (630). The response may include interactions of the user 210 with the memory words, substance use words, and/or neutral words. The one or more processors may provide a visualization instruction to the user 210 (635). The visualization instruction may prompt the user 210 to think of future events occurring for the user 210. The one or more processors may then receive event information (640). The event information may relate to the future event and may be input by the user 210. The one or more processors may provide a prompt for the event (645). The prompt may be based on the event information and provided following, before, or during performance of an activity (e.g., selection activity). The one or more processors may then receive a response based on the prompt (650). The one or more processors may present a subsequent substance use word instruction and/or memory word instruction to the user and then a subsequent visualization instruction including a prompt, and this cycle may be repeated one or more times during a period of 1-24 hours, 1-7 days, 1-4 weeks, 1-12 months, or longer than a year. The response may be based on a performance of the user in response to receiving the prompt. The one or more processors can then update the profile based on the responses (655). The responses may include scores based on the user's 210 performance.

### 11. Dynamic content delivery

In some implementations, the system can include dynamic content delivery that transmits requests to the user device.

For example, the activity selector 150 may select the activity based on an optimal time to send the user 210 the activity. The activity selector 150 is configured to select activities and instruct the session handler 140 to send the instruction 215 with "just-in-time-interventions" (JITIs) to reinforce training provided by the activities. For example, the activity selector 150 may select both an activity and a time to send the user 210 sessions based on the user profile 175 and past sessions stored in the database 170. The activity selector 150 may also select a length of time the activity is performed based on the user profile 175 and past sessions stored in the database 170.

In some embodiments, the activity selector 150 can select and/or initiate an activity based on physiological changes of the user as measured by a wearable technology. For example, responsive to a biological signature indicating an increased risk of substance use as measured by a wearable technology (e.g., smart watch, smart phone) of the user, the application 125 may initiate a session 210 to address the maladaptive behaviors related to substance use. The activity selector 150 may select an activity based on the biological signature provided by the wearable technology. Thus, the application 125 and activity selector 150 can adjust the timing and type of instructions and activities based on physiological changes of the user as measured by a wearable technology in order to provide personalized interventions and activity schedules to address substance use.

In some embodiments, the session handler 140 may provide an instruction in response to receiving a request from the user 210 indicating an onset of behavior associated with the substance use. For example, the user 210 may feel an urge to engage in substance use, and provide a request to the session management service 105 to initiate a session. The session handler 140 can then initiate instructions for activities to address the maladaptive behaviors associated with substance use in the user 210. These activities may include, for example, selection activities and visualization activities to reinforce learnings of the user 210 in past sessions. The sessions may be provided just in time and as requested by the user 210.

In some embodiments, the session handler 140 provides sessions in predetermined time periods. Based on the user profile 175, the session management service 105 can determine a schedule of activities for the user 210. For example, the session management service 105 can set a selection activity to send at a first time, and then set a visualization activity to send a day later. The schedule may be adjusted based on responses received from the user 210 based on interactions with the activities. The session handler 140 may also determine a time to provide instructions to the user 210.

In some embodiments, the session handler 140 provides sessions based on JITIs. For example, the session handler 140 can provide sessions at determined times to the user 210 based on the user profile 175, response scores of the user 210, and by user 210 request. The session handler 140 may be able to determine an optimal time to provide the user 210 with sessions. For example, the session handler 140 may determine a time of day at which a response is more likely than other times of day as determined by the user's previous activity. The session handler 140 may then determine to provide sessions to the user 210 consistently at the time of day at which a response is more likely.

### B. Systems and Methods for Ameliorating Substance Use in Users via Administration of Digital Therapeutics

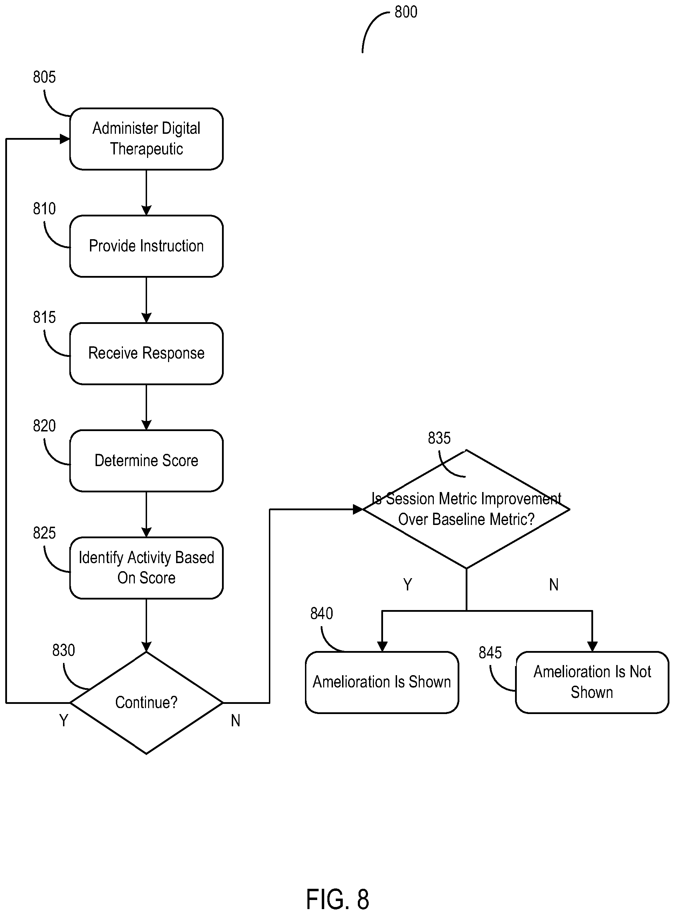

FIG. 8 depicts a flow diagram of a method 800 for ameliorating substance use in a user. The method 800 may be performed by any components of the system 100, such as the session management service 105, the user device 110, or the user 210, among others. Under the method 800, one or more processors can administer a digital therapeutic to a user (805). The digital therapeutic can be targeted at addressing substance use in the user. The one or more processors can provide an instruction to the user (810). The instruction may instruct the user to perform an activity. The one or more processors can receive a response (815). The response can be based on a performance of the user during the activity. The one or more processors can determine a score (820). The score can be based on the response. The one or more processors identify an activity based on the score (825). The score can dictate a subsequent activity for the one or more processors to provide to the user. The one or more processors can decide whether or not to continue with the digital therapeutic (830). This can be based on an efficacy of the intervention or a length of time of the intervention, among others. The one or more processors can determine whether the session metric shows an improvement over the baseline metric (835). The method 800 may include determining that an amelioration is shown when the session metric is determined to have improved over the baseline metric (840). The method 800 may include determining that no amelioration is shown when the session metric is determined to have not improved over the baseline metric (845).

In further detail, the method 800 may include administering a digital therapeutic to the user. The digital therapeutic may be associated with a user (e.g., the user 210) having a SUD. The SUD may be a result of genetics, brain chemistry (e.g., changes in neurotransmitter levels like dopamine), mental health disorders (e.g., anxiety, depression, or attention deficit hyperactivity disorder (ADHD)), personality traits (e.g., impulsive behavior), coping mechanisms (e.g., lacking effective coping strategies), or early exposure (e.g., during adolescence when the brain is still developing), among others. The impact of SUDs on the user can include physical health effects (e.g., cardiovascular diseases, lung disease, etc.) as well as mental health effects (e.g., depression, anxiety, etc.).

The user may be of any demographic or trait, such as by age (e.g., an adult (above age of 18) or late adolescent (between ages of 18-24)) or gender (e.g., male, female, or non-binary), among others. In at least partial concurrence with the time instance or the sessions during which the user is to perform activities, the user may be on a medication to address the pathology. For example, the medication may include naloxone, naltrexone, nalmefene, samidorphan, nalorpine, levallorphan, nalodeine, alvimopan, methylnatrexone, naloxegol, acamprosate, disulfiram, topiramate, gabapentin, methadone, buprenorphine, lofexidine, or a GLP-1 agonist, among others. The medication may be of an effective amount (e.g., dosage or frequency), which may be sufficient to effect positive outcomes or reduction in symptoms (e.g., reduction in weight). The efficacy of the medication in terms of addressing the user's SUD may be improved or enhanced from the concurrence with the intervention performed and monitored via the digital therapeutic application.

In some embodiments, the users of the digital therapeutics application may be exposed to or provided with the sessions to be performed by the user via the digital therapeutics application (e.g., the application 125 or the Study App described herein). As discussed herein, the sessions may include repeated cycles of selection activities and visualization activities as described herein. The second group may include another set of users for a control group. The users of the second group may be exposed to or provided with a control therapy (sometimes herein referred to as a placebo or standard treatment).

The control therapy may exclude the activities aimed at addressing the SUD and to be performed via the digital therapeutics application. In some embodiments, the control therapy may include an intervention performed via an application on a computing device that is inert with respect to the SUD or related symptoms. In some embodiments, the control therapy may include an inert version of the intervention without any association to SUD. The inert version of the intervention may lack proper generation of the intervention per user.

In some embodiments, the control therapy may include a care-as-usual (CAU) treatment regimen. The CAU treatment regimen may lack the digital therapeutics application described herein. For example, the CAU treatment regimen may have the user receive medication for the SUD on a scheduled basis (e.g., one to three times a day), and may include assessments of the SUD conducted in person or via a web application to evaluate symptoms. Baseline metrics (e.g., the metric before the intervention) may be obtained (e.g., via a computing device) from users of both the treatment group and the control group (e.g., receiving an inert version or CAU).

The method 800 may include providing instructions to the user (710). The instructions may be related to at least one of or a combination of the selection activities and visualization activities. For example, repeated cycles of an instruction on substance use words and/or memory words and/or neutral words, and an instruction on information regarding a future event. The user may also be prescribed a medication for substance use. The user may be in treatment for substance use at least in partial occurrence with the method 800.

The method 800 may include receiving a response (815). The response can be an interaction of the user with the activity. For example, the response can include interactions of the user with words presented on a user device of the user, instances of the user withholding selections with the words, and instances of the user providing information on future events.

The method 800 can include determining a score based on the response (820). The score can be based on a number of correct interactions, a percentage of correct interactions, a speed of selections, or any combination thereof. The correct interactions indicate a number of times that the user correctly selected or refrained from selecting the words. For example, for a selection activity, a correct interaction may be the user selecting a neutral word, refraining from selecting a substance use word, and/or refraining from selecting a memory word. As another example, for a memory quiz, a correct interaction may be the user selecting a memory word and refraining from selecting a neutral word. The speed of selections may indicate a time between the word appearing on the user device, and the user selecting the word.

The method 800 can include identifying an activity based on the score (825). The score can indicate a performance and a progress for the user. The score can satisfy a threshold and indicate to increase a number of words and/or increase a difficulty of a subsequent activity. The score can also indicate a type of activity to provide to the user, such as selection activity or visualization activity.

The baseline measure may be (e.g., by a computing device) obtained prior to performing any activities of the intervention performed by the user via a digital therapeutic application (e.g., the application 125 or the Study App described herein). The baseline measure may indicate the condition of the user with respect to the severity of the SUD. The baseline metric may include, for example, substance usage frequency, diagnostic and statistical manual of mental disorders, 5^{th} edition (DSM-5) diagnosis, Obsessive Compulsive Drug Use scale (OCDUS) metric, Drug Abstinence Self-Efficacy Scale (DASES) metric, Modified Visual Analogue Scale (MVAS) rating, self-reported substance use, Opioid Substitution Treatment Quality of Life (OSTQOL) scale, Recovery Empowerment Scale, Perceived Stress Scale, impact on daily functioning, tolerance, withdrawal, or duration of use, among others. Before performance of the intervention, the user may have an initial baseline metric satisfying a baseline threshold. The threshold may delineate, define, or identify a value for the initial metric at which the user is identified to be suitable or eligible for performance of the activities via the digital therapeutic application. The initial metric may be, for example, the DSM-5 satisfying 4 or more criteria.

The session metric may be associated with the user (e.g., the user 210) with respect to the SUD. The session metric may be obtained (e.g., by a computing device) subsequent to performing at least one of the time instances or sessions during which an activity is to be performed by the user via the digital therapeutic application. The baseline measure may indicate the condition of the user with respect to the severity of the SUD and the associated impact on the user's daily life and activities, as a result of the performance of the intervention. The session metric may include, for example, substance usage frequency, diagnostic and statistical manual of mental disorders, 5^{th} edition (DSM-5) diagnosis, Obsessive Compulsive Drug Use scale (OCDUS) metric, Drug Abstinence Self-Efficacy Scale (DASES) metric, Modified Visual Analogue Scale (MVAS) rating, self-reported substance use, Opioid Substitution Treatment Quality of Life (OSTQOL) scale, Recovery Empowerment Scale, Perceived Stress Scale, impact on daily functioning, tolerance, withdrawal, or duration of use, among others. The session metric may be of the same type of measurement as the baseline metric. The session metrics may be obtained from users of both the treatment group and the control group.

The method 800 may include identifying or determining whether to continue (830). The determination may be based on the set length (e.g., days, weeks, or years) of the trial, a set number of time instances during which to perform one or more activities, a set amount of time performing activities, or a set number of activities to be provided to the user. For example, the set number of time instances may range between 1 week to 6 months, relative to the obtaining of the baseline metric. For example, the digital therapeutic can be administered at least daily, every other day, every 3 days, or weekly. When the amount of time from the obtaining of the baseline metric exceeds the set length, the determination may be to stop providing additional activities.

In contrast, when an amount of time has passed from when the baseline metric was obtained has not exceeded the set length, the determination may be to continue providing additional interventions and repeat from (805). The selection or modification of the instruction for the next time instance or session may be based on the performance of the activity in the previous time instance or session. In some embodiments, the next instruction may be modified based on response data from the user while performing the activity in the prior time instance or session. In some embodiments, the next instruction may be modified based on a score determined from the performance of the activity in the prior time instance or session. The instruction may be modified, for example, by order of performance (e.g., with respect to other interventions), a time length to complete the intervention, and a difficulty of performing the intervention, among others. The selection or modification of the instruction for the next time instance or session may be independent from the performance of the intervention in the previous time instance or session.

The method 800 may include identifying or determining whether the session metric is an improvement over the baseline metric (835). The improvement may correspond to the reduction in substance use or the intensity of the side effects related to the SUD. The improvement may be shown when the session metric is increased compared to the baseline metric by a first predetermined margin or when the session metric is decreased compared to the baseline metric by a second predetermined margin. The margin may identify or define a difference in value between the baseline and session metrics at which to determine that the user shows reduction in the intensity of the side effects related to the SUD. Whether the improvement is shown by increase or decrease may depend on the type of metric used to measure the user with respect to the SUD. The margin may also depend on the type of metric used, and may in general correspond to the difference in value showing noticeable difference with respect to the SUD, or showing a statistically significant result in the difference in the values between the baseline and session metrics.

In some embodiments, at least one of the first predetermined margin or the second predetermined margin may be calculated, generated, or otherwise determined (e.g., by a computing device). The first predetermined margin or the second predetermined margin may be based on the baseline and session metrics obtained from the treatment group or the control group of users. The margin may correspond to a difference between a change in baseline and session metrics in the treatment group versus a change in baseline and session metrics in the control group (e.g., receiving the control therapy) that is statistically significant. The difference may be based on any number of statistical tests, such as a t-test, a z-test, chi-squared test, among others.

The method 800 may include determining that amelioration is shown when the session metric is determined to be an improvement over the baseline metric (840). In some embodiments, the amelioration may be determined (e.g., by the computing system or a clinician examining the user) to occur when the DSM-5 metric is decreased from the baseline DSM-5 metric by the first predetermined margin. In some embodiments, the amelioration may be determined to occur when the session OSTQOL metric is decreased from the baseline OSTQOL metric by the second predetermined margin. In some embodiments, the amelioration may be determined to occur when the session withdrawal metric is decreased from the baseline withdrawal metric by the second predetermined margin. In some embodiments, the amelioration may be determined to occur when the session impact on daily life percentage metric is decreased from the baseline impact on daily life percentage metric by the first predetermined margin. The substance use can be decreased in the user following administration of the digital therapeutic.

The method 800 may include determining that amelioration is not shown when the session metric is determined to be not an improvement over the baseline metric (845). In some embodiments, the amelioration may be determined (e.g., by the computing system or a clinician examining the user) to not occur when the DSM-5 metric is not decreased from the baseline DSM-5 metric by the first predetermined margin. In some embodiments, the amelioration may be determined to not occur when the session OSTQOL metric is not decreased from the baseline OSTQOL metric by the second predetermined margin. In some embodiments, the amelioration may be determined to not occur when the session withdrawal metric is not decreased from the baseline withdrawal metric by the second predetermined margin. In some embodiments, the amelioration may be determined to not occur when the session impact on daily life percentage metric is not decreased from the baseline impact on daily life percentage metric by the first predetermined margin.

### Example 1: Efficacy of Cognitive Activities in a Digital Therapeutics to Target SUDs

In one example, the CT-102 mobile app (e.g., the application 125) is given to individuals with a self-reported diagnosed moderate to severe opioid use disorder (OUD) according to a diagnostic and statistical manual of mental disorders, 5^{th} edition (DSM-5) criteria. DSM-5 includes a list of criteria and identifies an individual with moderate to severe OUD by the individual fulfilling 4-5 criteria for moderate OUD, and 6 or more for severe OUD. The treatment is about 2-52 weeks long. This study is a single-arm study to examine the feasibility and acceptability of an abbreviated CT-102 app for participants aged 18 years and older with an OUD. There are at least 20 participants in the single-arm.

At least 20 participants are enrolled to achieve at least 10 completers, based on an expected Early Termination (ET) rate of 33%. This estimate is based on an approximation, reflecting an established ET of 50% in Randomized Control Trials with treatment lengths of 2 to 52 weeks. There is a sub-cohort within this arm of approximately 5-10 participants who voluntarily consent to be a part of User Experience Research (UXR) surveys and interviews as specified.

The users will engage in activities as disclosed herein, including the selection activities, visualization activities and memory quiz. The users will be evaluated based on a number of clinical endpoints provided below.

Screening Period: All participants are screened for up to 7 days. Assessments during this period are performed according to the Schedule of Activities and Assessments (SoA). Participants are considered eligible if they meet all inclusion criteria and no exclusion criteria, based on investigator assessment. Screening and a Baseline Visit can occur on the same day.

Baseline Visit: At the end of the Screening Period, eligible participants are enrolled. Baseline evaluations are performed according to the SoA. Participants download and activate a Study App (e.g., abbreviated CT-102) onto their personal primary iPhone or Android smartphone. The participants are directed to access and engage with the Study App daily.

Engagement Period: Participants interact with the Study App daily and complete activities as directed by the Study App. During the Engagement Period, participants conduct oral fluid drug tests about 2 times each week. These tests are performed in support of the treatment mechanisms delivered by the Study App and are not considered to be study endpoints. Oral fluid drug testing includes remote administration of point-of-care testing twice weekly and remote provision of an additional oral fluid specimen for confirmatory laboratory testing (transmitted by mail) once weekly. Participants undergo evaluations as described in the SoA. Approximately 6 participants undergo a virtual interview towards the end of Week 2 (Day 9 to Day 15). Approximately 10 participants take part in weekly user engagement surveys.

Follow-up Period: At the end of the engagement, participants undergo post-treatment follow up for one week. Participants undergo evaluations as described in the SoA. Participants who have opted into the UXR will participate in the final UXR interview.

Primary Endpoints:
- Engagement Levels
   ∘ Number of App engagement days during engagement period
   ∘ Number of lessons completed during the engagement period
   ∘ Number of DiNaMo^{™} sessions completed during the engagement period
- Therapeutic Alliance
   ∘ Change in Mobile Agnew Relationship Measure (mARM) during engagement period
- Change in Mobile Agnew Relationship Measure (mARM) during engagement period Cognitive Functions
   ∘ Change during engagement period in delayed discounting metric derived from the Monetary Choice Questionnaire
   ∘ Change during engagement period in drug demand metric derived from the Hypothetical Purchase Task
   ∘ Change during engagement period in objective working memory function as measured by the Digit Span task
   ∘ Change during engagement period in subjective working memory function as measured by the Meta-Memory Questionnaire
   ∘ Change during engagement period in subjective inhibitory control as measured by the Barratt Impulsiveness Scale (BIS-11)
   ∘ Maximum working memory challenge condition achieved through DiNaMo during the engagement period
   ∘ Active working memory challenge condition in DiNaMo during engagement period
   ∘ Maximum response latency challenge condition achieved through DiNaMo during the engagement period
   ∘ Active response latency challenge condition in DiNaMo during engagement period
- App Usability and Perception
   ∘ Patient Global Impression of Change (PGI-C single item) during engagement period
- Opioid Use and Craving Management
   ∘ Change during engagement period in the Obsessive-Compulsive Drug Use Scale [OCDUS]
   ∘ Change during engagement period in Drug Abstinence Self-Efficacy (Drug Abstinence Self-Efficacy Scale [DASES])
   ∘ Change during engagement period in the Modified Visual Analogue Scale (MVAS) rating for cravings
- Participant Feedback
   ∘ Customer Satisfaction (CSAT) (single item) during engagement period
   ∘ Digital Intervention Barriers Scale (DIBS-7) during engagement period
- Change during engagement period in Recovery Empowerment Scale

### Change during engagement period in Perceived Stress Scale

### Exploratory Endpoints:

- Change during engagement period in self-reported opioid use with the Timeline Follow-Back (TLFB) Procedure
- Change during engagement period in self-reported total substance use with the TLFB Procedure
- Change during engagement period in Recovery Empowerment Scale
- Change during engagement period in Perceived Stress Scale
- Change during engagement period in Opioid Substitution Treatment Quality of Life (OSTQOL)

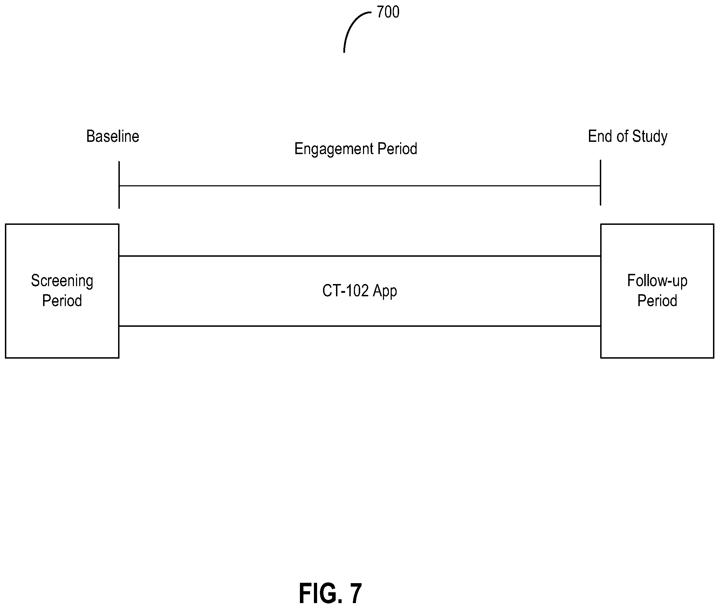

**Correlations between** User Engagement Metrics, Perceived Treatment Outcome (PGI-C), User Satisfaction (CSAT), as well as treatment-related change in OCDUS, DASES, MVAS, Delay Discounting (Monetary Choice Questionnaire), cognitive measures (BIS-11, Meta-Memory Questionnaire, Digit Span) and TLFB Schedule of Activities and Assessments (SoA). A study performed using the CT-102 mobile app in adults (18+ years) with substance use-related disorders can follow a study schedule 700 as shown in FIG. 7.

### Screening Period

- All participants enter a Screening Period of up to 7 days to determine eligibility for enrollment. Assessments during this period are performed according to the Schedule of Activities and Assessments (SoA).
- A participant may complete Screening and Baseline Visits on the same day if all required assessments have been completed per the protocol.

### Baseline visit (Day 1)

- At the end of the Screening Period, eligible participants are enrolled.
- Participants also undergo baseline evaluations as described in the Schedule of Activities and Assessments.
- Site personnel assist eligible participants to download and install the Study App onto their personal primary smartphone. The Study App is activated using a unique activation code.
- Participants are directed to access and engage with the Study App daily.
- A participant complete Screening and Baseline Visits on the same day if all required assessments have been completed per the protocol.

### Engagement Period

- Participants interact with the Study App daily and complete activities as directed by the Study App.
- During the Engagement Period, participants conduct oral fluid drug tests about 2 times each week. These tests are performed in support of the treatment mechanisms delivered by the Study App and are not considered to be study endpoints. Oral fluid drug testing includes remote administration of point-of-care testing twice weekly and remote provision of an additional oral fluid specimen for confirmatory laboratory testing (transmitted by mail) once weekly.
- Participants undergo evaluations as described in the SoA
- Approximately 6 participants undergo a virtual interview towards the end of Week 2
- Approximately 10 participants take part in weekly user engagement surveys

### Follow-Up Period (1 week)

- At the end of the engagement, participants will undergo post-treatment follow up. Participants will undergo evaluations as described in the SoA.
- Participants who have opted into the UXR component participate in the final UXR interview.

At least 20 participants are enrolled in this study. Approximately 10 sites will participate in the study.

### Study Assessments

### Monetary Choice Questionnaire

The Monetary Choice Questionnaire (MCQ) is a self-report assessment tool used to assess delay discounting behavior, which measures the tendency to devalue rewards as the delay to receiving them increases. Through a series of 27 hypothetical decision-making prompts, participants choose between smaller, immediate rewards and larger, delayed rewards. The rate at which individuals discount future rewards indicates their preference for immediate gratification versus delayed benefits, providing insights into decision-making processes related to financial decisions, impulsivity and health behaviors. This delay discounting rate can be computed from MCQ responses.

### Hypothetical Purchase Task

The Hypothetical Purchase Task (HPT) is a tool used to measure hypothetical drug demand for opioids and other substances. In the HPT, participants report the quantity of a drug they would purchase at 9 increasing prices. This method helps researchers create demand curves and assess drug demand behaviors. Studies have found correlations between these demand measures and clinical variables like substance use and treatment outcomes, including prediction of future opioid use in patients with OUD.

### Barratt Impulsiveness Scale (BIS-11)

The BIS-11 was designed to assess impulse control across attentional (e.g., difficulty concentrating), motor (e.g., acting on auto-pilot), and planning-related (e.g., failure to plan ahead) domains. It was intended to be an improvement over previous versions of the BIS. The BIS-11 has previously demonstrated treatment-related change in response psychological interventions in patients with OUD.

It ranks 30 items using 4-point ratings (1 = never/rarely, 2 = occasionally, 3 = often, 4 = almost always/always). The BIS-11 has 3 sub-scales:
Attentional Impulsiveness assesses task-focus, intrusive thoughts, and racing thoughts.
Motor Impulsiveness assesses tendency to act on the spur of the moment and consistency of lifestyle.
Non-planning Impulsiveness assesses careful thinking and planning and enjoyment of challenging mental activities.

### Drug Abstinence Self-Efficacy Scale (DASES)

DASES is a 20-item self-report survey that measures confidence in one's ability to abstain from using drugs in specific situations. The DASES is a psychometrically-validated adaptation of the Alcohol Abstinence Self-Efficacy Scale.

### Customer Satisfaction (CSAT) Single Item

The CSAT is a single-item rating of customer satisfaction with a product or service. This survey is intentionally designed to be simple, encouraging user engagement and authentic feedback. Participants are asked to rate their satisfaction on a 5-point scale, where 1 represents "very dissatisfied" and 5 represents "very satisfied." This format allows for a straightforward, quantitative assessment of customer satisfaction, providing valuable insights into the effectiveness and user-friendliness of a digital interaction or intervention.

### Meta-Memory Questionnaire (MMQ)

The Meta-Memory Questionnaire (MMQ; also referenced as the Multifactorial Memory Questionnaire) is a self-report tool designed to assess 3 subjective dimensions of memory: overall satisfaction with one's memory (MMQ-Contentment), perception of everyday memory ability (MMQ-Ability), and use of memory strategies and aids (MMQ-Strategy). The MMQ focuses on clinically relevant aspects of memory, including memory-related affect, and avoids items unrelated to everyday functioning. The questionnaire includes a total of 57 self-report items and should take approximately 10 minutes to complete. The MMQ has been determined to have excellent psychometric properties and has additionally demonstrated responsivity to brief cognitive training interventions in clinical populations.

### Digital Intervention Barriers Scale (DIBS-7)

The DIBS-7 identifies a 10-item unidimensional scale that captures commonly faced barriers in digital mental health interventions. The DIBS-7 has demonstrated strong criterion-related validity, with mean scores robustly correlating with established variables related to digital mental health intervention (DMHI) engagement, including treatment expectations, behavioral indicators of engagement, and satisfaction. These associations were stronger than those observed for user characteristics related to engagement, suggesting broad applicability across different user demographics. The DIBS-7 was further shown to significantly moderate treatment response, highlighting the DIBS-7's relevance in assessing factors that impact DMHI effectiveness.

### Mobile Agnew Relationship Measure (mARM)

The mARM is a validated patient-rated scale that assesses the DWA with mobile health interventions for mental health conditions. It was adapted from the well-validated Agnew Relationship Measure and consists of 25 items that are rated on a 7-item scale that ranges from "Strongly Disagree" to "Strongly Agree."

### System Usability Scale (SUS)

The System Usability Scale (SUS) is a widely used scale that has been used to quantify the usability of many software and hardware products. The SUS score is presented as a percentage, ranging from 0 to 100. A perfect score of 100% signifies flawless usability and an exceptional user experience.

### Patient Global Impression of Change (PGI-C)

The Patient Global Impression of Change (PGI-C) is a self-reported, 7-point scale depicting a patient's rating of overall improvement. PGI-C has been validated in a number of other episodic diseases against well-established measures of pain intensity, pain interference in daily life, and treatment effectiveness. Importantly, the change from baseline, not from last visit, is evaluated.

### Obsessive Compulsive Drug Use Scale (OCDUS)

The Obsessive Compulsive Drug Use Scale (OCDUS) measures 3 factors: thoughts about the substance and interference, desire and control, and resistance to thoughts and intention.

### Self-Reported Substance Use via Timeline Follow-Back (TLFB)

TLFB retrospectively collects data on substance use for each day of the reporting period, and thus provides detailed information on use patterns. The TLFB was originally developed for the assessment of self-reported alcohol use (Sobell & Sobell, 1992), but the procedure was later adapted to support evaluation of other substance use, including opioids, and has been validated for this purpose.

### Recovery Empowerment Scale

The Recovery Empowerment Scale (RES) is a self-report instrument with 20 items designed to assess empowerment in individuals recovering from substance use disorders. Seven items are adapted from the Women in Recovery Empowerment Scale. The RES evaluates four dimensions of empowerment: emotional, cognitive, behavioral, and relational. Specifically, it includes four items for emotional empowerment, four for cognitive, three for behavioral, and nine for relational. Respondents rate items on a seven-point Likert scale from "Almost never" to "Almost always," with higher scores indicating greater empowerment. The scale is applicable to both men and women, offering a thorough and reliable measure of empowerment across different populations.

### Perceived Stress Scale

The Perceived Stress Scale is a 14-item instrument designed to measure the degree to which situations in one's life are appraised as stressful. The scale also includes a number of direct queries about current levels of experienced stress.

### Brief Trauma Questionnaire (BTQ)

The BTQ is a 10-item self-report questionnaire designed to assess traumatic exposure according to *DSM-IV* but specifically including only life threatening and serious injury because of the difficulty of accurately assessing subjective response.

The BTQ was initially created to evaluate traumatic exposure in line with DSM-IV guidelines, focusing solely on Criterion A.1 (life threat/serious injury) due to the challenges of accurately measuring Criterion A.2 (subjective response) in a brief self-report format. Since Criterion A.2 has been removed from the PTSD diagnostic criteria in DSM-5, the BTQ now offers a comprehensive assessment of Criterion A.

### Opioid Substitution Treatment Quality of Life Scale (OSTQOL)

The Opioid Substitution Treatment Quality of Life Scale (OSTQOL) is a specialized instrument developed to assess the quality of life (QOL) in patients undergoing opioid substitution treatment (OST). This scale was created through a rigorous process involving focus groups with OST patients to identify key QOL themes. The final scale includes 38 items, across six subscales that capture various aspects of QOL, some uniquely relevant to persons with OUD: Personal Development, Mental Distress, Social Contacts, Material Well-being, Satisfaction with Opioid Substitution Treatment, and Experiences of Stigma and Discrimination. This instrument has demonstrated good internal consistency reliability, strong convergent and discriminant validity, as well as good responsivity to treatment in largescale trials within the OUD patient population. The OSTQOL is a multidimensional tool designed to minimize respondent and administrator burden while providing a comprehensive assessment of QOL in OST patients.

### Modified Visual Analogue Scale (MVAS)

The modified VAS for craving is a 2D representation to assess both the intensity and the frequency of cravings (expressed by the percentage of the time in which craving is experienced during the day). Unlike the standard VAS, which typically consists of a single line marked with endpoints indicating "no pain" and "worst pain imaginable," the MVAS includes additional markers or descriptors along the scale allowing participants to more accurately represent their pain levels by distinguishing between mild, moderate and severe pain. The use of MVAS reduces ambiguity and improves the reliability of pain assessments.

### Columbia-Suicide Severity Rating Scale (C-SSRS)

The Columbia-Suicide Severity Rating Scale (C-SSRS) is a suicidal ideation and behavior rating scale that rates an individual's degree of suicidal ideation on a scale, ranging from "wish to be dead" to "active suicidal ideation with specific plan and intent and behaviors." This evaluation consists of a baseline evaluation that assesses the lifetime experience of the participant with suicide events and suicidal ideation and a postbaseline evaluation that focuses on suicidality since the last trial visit.

### UXR Interview

Some participants will participate in video-conference-based qualitative user interviews with members of the Click Therapeutics Product Development team. Approximately 6 participants will take part in a UXR interview at Week 2 (Day 9 to Day 15) and 10 participants will take part in a Final Visit interview. These interviews will gather additional information about the user's experience with the App, such as favorite App features, usability of the features, challenges related to the intervention or any other feedback from regularly interacting with the App.

### User Engagement Surveys

The User Engagement Surveys will be developed to understand a participant's experience with the Study App during the engagement phase. The questionnaires will ask questions related to perceived enjoyment, challenges and related user experience. These questionnaires will be completed electronically by the participant once per week. They will take approximately 10 minutes to complete.

### Example 2: Clinical Evaluation of the Safety and Effectiveness of Digital Therapeutics Application

This study evaluates the effectiveness and safety of CT-102 as adjunctive therapy in participants 18 years of age and older with OUD who are treated with an FDA approved medication for OUD (MOUD). The treatment interval is 2-52 weeks. The digital therapeutics application is evaluated as an adjunct treatment for OUD. The users will engage in activities as disclosed herein, including the selection activities, visualization activities and memory quiz. The users will be evaluated based on a number of clinical endpoints provided below.

The users of the CT-102 application are evaluated against one or more controls for statistically significant differences in the clinical primary and secondary endpoints described herein. The one or more controls include control groups who do not engage with the CT-102 group, users who do not engage in one or more selection activities, visualization activities or memory quiz activities. The changes from baseline in clinical primary and secondary endpoints are evaluated for the CT-102 and control groups and the values are evaluated for clinically significant changes for the CT-102 group versus control groups.

Study Objectives: The purpose of the study is to evaluate the effectiveness and safety of CT-102 as adjunctive therapy in participants 18 years of age and older with OUD on MOUD over a treatment interval.

Primary Endpoints: Biochemically verified non-prescription opioid abstinence, as defined by ≥80% negative samples over the evaluation period.

### Secondary Endpoints:

- Change in self-reporting of non-opioid substance use (timeline follow-back procedure) during engagement period
- Change in self-reporting of opioid substance use (timeline follow-back procedure) during engagement period
- Change in Recent Overdose Experiences Scale (RODES) during engagement period
- Change in Personal Opioid Overdose Risk Survey (PORS) during engagement period
- Change in Opioid Substitution Therapy Quality of Life (OSTQOL) during engagement period
- Change in Short Form Health Survey (SF-36) during engagement period
- Change in Obsessive Compulsive Drug Use Scale (OCDUS) during engagement period
- Change in Drug Abstinence Self-Efficacy Scale (DASES) during engagement period
- Change in biochemically verified non-opioid substance use during engagement period
- Change in Retention in MOUD as reported by study participant with adequate clinical documentation (to be provided to study team upon request) during engagement period
- To evaluate overall improvement via Patient Global Impression of Change (PGI-C) during engagement period
- Durability of effect will be measured by biochemically verified non-prescription opioid abstinence during engagement period

### Exploratory Endpoints:

- Degree of participant engagement with the investigational digital therapeutic as measured by key engagement metrics
- Durability of effect will be measured by self-reporting of opioid substance use (timeline follow-back procedure) during and/or after engagement period
- Durability of effect will be measured by self-reporting of non-opioid substance use (timeline follow-back procedure) during and/or after engagement period

### Safety Endpoints:

- Frequency and severity of AEs, serious AEs, and discontinuation from the study due to AEs
- Incidence of suicidal behavior and ideation, as assessed by the Columbia-Suicide Severity Rating Scale (C-SSRS)

Study Design: This is a multi-center, randomized, double-blind, digitally controlled study to evaluate the effectiveness and safety of CT-102 as an adjunctive to treatment with MOUD for OUD in participants 18 years of age and older.

### Screening Period:

- All participants enter a Screening Period of up to 14 days to determine eligibility for enrollment
- During the screening period, participants are asked to complete various assignments within the onboarding software module to confirm digital literacy and interest in the study
- Assessments during this period is performed according to a Schedule of Activities and Assessments (SoA)

Run in Period: Participants then undergo a 28-day Run-in Period designed to establish a baseline of use.

Baseline Visit: After completing the 28-day Run-in Period, the investigator ensures the participant continues to meet all inclusion and no exclusion criteria. Participants are randomized to CT-102 or a Digital Control on Day 1.

### Treatment Period:

- Participants follow protocol treatment assignment for up 365 days
- Participants interact with the Study App daily and complete tasks as directed by the Study App
- During the Treatment Period, participants conduct oral fluid drug tests ~2 times each week. Between Treatment Periods, a responder with greater than or equal to sixteen (16) out of twenty (20) negative samples during this period is considered abstinent
- Oral fluid drug testing includes remote administration of point-of-care testing twice weekly and additional remote oral fluid specimens for confirmatory laboratory testing (transmitted by mail) twice weekly.
- Results from biospecimens are additionally be cross-referenced against self-reported opioid use (assessed via the timeline follow-back procedure)

Follow-Up Period: After completing assigned treatment, participants are monitored for up to 12 weeks and undergo post-treatment follow up. Participants undergo evaluations as described in the SoA.

Statistical Analysis: The equivalence of treatment conditions regarding key baseline variables is assessed. This involves comparisons of treatment conditions on demographics and baseline levels of potential treatment moderators (e.g., gender and age). Following the intent-to-treat principle, all randomized participants are included in the analyses. The null hypothesis for the primary endpoint is that the success proportion between the two arms is not different and the alternative hypothesis is that they are different. Test of proportions are used to assess this hypothesis. The primary goal is to yield a stable estimate of the effect size and adjust the sample size to show statistically significant differences between treatment groups for the primary endpoints.

### C. Network and Computing Environment

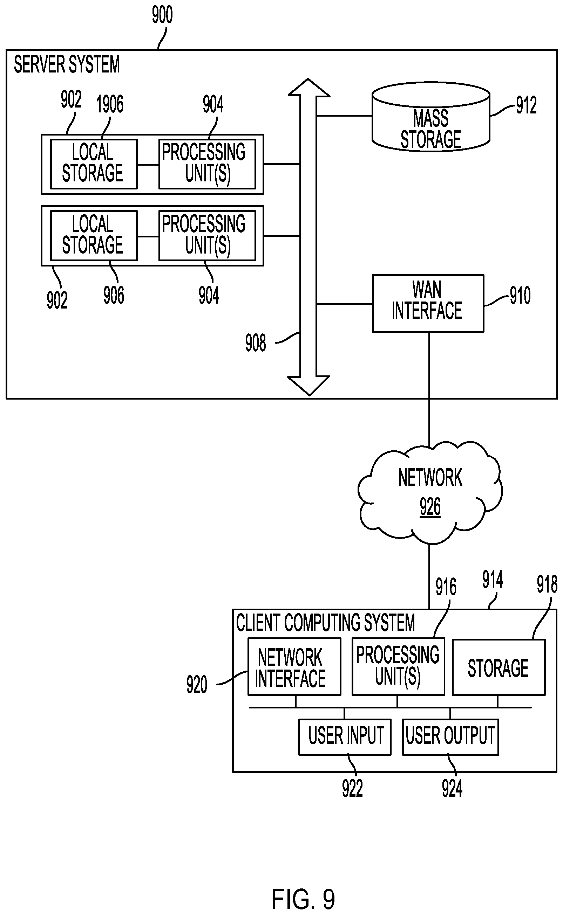

Various operations described herein can be implemented on computer systems. FIG. 9 shows a simplified block diagram of a representative server system 900, client computing system 914, and network 926 usable to implement certain embodiments of the present disclosure. In various embodiments, server system 900 or similar systems can implement services or servers described herein or portions thereof. Client computing system 914 or similar systems can implement clients described herein. The system 900 described herein can be similar to the server system 900. Server system 900 can have a modular design that incorporates a number of modules 902 (e.g., blades in a blade server embodiment); while two modules 902 are shown, any number can be provided. Each module 902 can include processing unit(s) 904 and local storage 906.

Processing unit(s) 904 can include a single processor, which can have one or more cores, or multiple processors. In some embodiments, processing unit(s) 904 can include a general-purpose primary processor as well as one or more special-purpose co-processors, such as graphics processors, digital signal processors, or the like. In some embodiments, some or all processing unit(s) 904 can be implemented using customized circuits, such as application specific integrated circuits (ASICs) or field programmable gate arrays (FPGAs). In some embodiments, such integrated circuits execute instructions that are stored on the circuit itself. In other embodiments, processing unit(s) 904 can execute instructions stored in local storage 906. Any type of processors in any combination can be included in processing unit(s) 904.

Local storage 906 can include volatile storage media (e.g., DRAM, SRAM, SDRAM, or the like) or non-volatile storage media (e.g., magnetic or optical disk, flash memory, or the like). Storage media incorporated in local storage 906 can be fixed, removable, or upgradeable as desired. Local storage 906 can be physically or logically divided into various subunits, such as a system memory, a read-only memory (ROM), and a permanent storage device. The system memory can be a read-and-write memory device or a volatile read-and-write memory, such as dynamic random-access memory. The system memory can store some or all of the instructions and data that processing unit(s) 904 need at runtime. The ROM can store static data and instructions that are needed by processing unit(s) 904. The permanent storage device can be a non-volatile read-and-write memory device that can store instructions and data even when module 902 is powered down. The term "storage medium" as used herein includes any medium in which data can be stored indefinitely (subject to overwriting, electrical disturbance, power loss, or the like) and does not include carrier waves and transitory electronic signals propagating wirelessly or over wired connections.

In some embodiments, local storage 906 can store one or more software programs to be executed by processing unit(s) 904, such as an operating system or programs implementing various server functions, such as functions of the system 900 or any other system described herein, or any other server(s) associated with system 900 or any other system described herein.

"Software" refers generally to sequences of instructions that, when executed by processing unit(s) 904, cause server system 900 (or portions thereof) to perform various operations, thus defining one or more specific machine embodiments that execute and perform the operations of the software programs. The instructions can be stored as firmware residing in read-only memory or program code stored in non-volatile storage media that can be read into volatile working memory for execution by processing unit(s) 904. Software can be implemented as a single program or a collection of separate programs or program modules that interact as desired. From local storage 906 (or non-local storage described below), processing unit(s) 904 can retrieve program instructions to execute and data to process in order to execute various operations described above.

In some server systems 900, multiple modules 902 can be interconnected via a bus or other interconnect 908, forming a local area network that supports communication between modules 902 and other components of server system 900. Interconnect 908 can be implemented using various technologies, including server racks, hubs, routers, etc.

A wide area network (WAN) interface 910 can provide data communication capability between the local area network (e.g., through the interconnect 908) and the network 926, such as the Internet. Other technologies can be used to communicatively couple the server system with the network 926, including wired (e.g., Ethernet, IEEE 802.3 standards) or wireless technologies (e.g., Wi-Fi, IEEE 802.11 standards).

In some embodiments, local storage 906 is intended to provide working memory for processing unit(s) 904, providing fast access to programs or data to be processed while reducing traffic on interconnect 908. Storage for larger quantities of data can be provided on the local area network by one or more mass storage subsystems 912 that can be connected to interconnect 908. Mass storage subsystem 912 can be based on magnetic, optical, semiconductor, or other data storage media. Direct attached storage, storage area networks, network-attached storage, and the like can be used. Any data stores or other collections of data described herein as being produced, consumed, or maintained by a service or server can be stored in mass storage subsystem 912. In some embodiments, additional data storage resources may be accessible via WAN interface 910 (potentially with increased latency).

Server system 900 can operate in response to requests received via WAN interface 910. For example, one of modules 902 can implement a supervisory function and assign discrete activities to other modules 902 in response to received requests. Work allocation techniques can be used. As requests are processed, results can be returned to the requester via WAN interface 910. Such operations can generally be automated. Further, in some embodiments, WAN interface 910 can connect multiple server systems 900 to each other, providing scalable systems capable of managing high volumes of activity. Other techniques for managing server systems and server farms (collections of server systems that cooperate) can be used, including dynamic resource allocation and reallocation.

Server system 900 can interact with various user-owned or user-operated devices via a wide-area network such as the Internet. An example of a user-operated device is shown in FIG. 9 as client computing system 914. Client computing system 914 can be implemented, for example, as a consumer device such as a smartphone, other mobile phone, tablet computer, wearable computing device (e.g., smart watch, eyeglasses), desktop computer, laptop computer, and so on.

For example, client computing system 914 can communicate via WAN interface 910. Client computing system 914 can include computer components such as processing unit(s) 916, storage device 918, network interface 920, user input device 922, and user output device 924. Client computing system 914 can be a computing device implemented in a variety of form factors, such as a desktop computer, laptop computer, tablet computer, smartphone, other mobile computing device, wearable computing device, or the like.

Processing unit 916 and storage device 918 can be similar to processing unit(s) 904 and local storage 906 described above. Suitable devices can be selected based on the demands to be placed on client computing system 914. For example, client computing system 914 can be implemented as a "thin" client with limited processing capability or as a high-powered computing device. Client computing system 914 can be provisioned with program code executable by processing unit(s) 916 to enable various interactions with server system 900.

Network interface 920 can provide a connection to the network 926, such as a wide area network (e.g., the Internet) to which WAN interface 910 of server system 900 is also connected. In various embodiments, network interface 920 can include a wired interface (e.g., Ethernet) or a wireless interface implementing various RF data communication standards such as Wi-Fi, Bluetooth, or cellular data network standards (e.g., 3G, 4G, LTE, etc.).

User input device 922 can include any device (or devices) via which a user can provide signals to client computing system 914; client computing system 914 can interpret the signals as indicative of particular user requests or information. In various embodiments, user input device 922 can include any or all of a keyboard, touch pad, touch screen, mouse or other pointing device, scroll wheel, click wheel, dial, button, switch, keypad, microphone, and so on.

User output device 924 can include any device via which client computing system 914 can provide information to a user. For example, user output device 924 can include display-to-display images generated by or delivered to client computing system 914. The display can incorporate various image generation technologies, e.g., a liquid crystal display (LCD), a light-emitting diode (LED) display including organic light-emitting diodes (OLED), a projection system, a cathode ray tube (CRT), or the like, together with supporting electronics (e.g., digital-to-analog or analog-to-digital converters, signal processors, or the like). Some embodiments can include a device such, as a touchscreen that functions as both an input and output device. In some embodiments, other user output devices 924 can be provided in addition to or instead of a display. Examples include indicator lights, speakers, tactile "display" devices, printers, and so on.

Some embodiments include electronic components, such as microprocessors, storage, and memory that store computer program instructions in a computer-readable storage medium. Many of the features described in this specification can be implemented as processes that are specified as a set of program instructions encoded on a computer-readable storage medium. When these program instructions are executed by one or more processing units, they cause the processing unit(s) to perform various operations indicated in the program instructions. Examples of program instructions or computer code include machine code, such as is produced by a compiler, and files including higher-level code that are executed by a computer, an electronic component, or a microprocessor using an interpreter. Through suitable programming, processing unit(s) 904 and 916 can provide various functionality for server system 900 and client computing system 914, including any of the functionality described herein as being performed by a server or client, or other functionality.

It will be appreciated that server system 900 and client computing system 914 are illustrative and that variations and modifications are possible. Computer systems used in connection with embodiments of the present disclosure can have other capabilities not specifically described here. Further, while server system 900 and client computing system 914 are described with reference to particular blocks, it is understood that these blocks are defined for convenience of description and are not intended to imply a particular physical arrangement of component parts. For instance, different blocks can be but need not be located in the same facility, in the same server rack, or on the same motherboard. Further, the blocks need not correspond to physically distinct components. Blocks can be configured to perform various operations, e.g., by programming a processor or providing appropriate control circuitry, and various blocks might or might not be reconfigurable depending on how the initial configuration is obtained. Embodiments of the present disclosure can be realized in a variety of apparatus including electronic devices implemented using any combination of circuitry and software.

While the disclosure has been described with respect to specific embodiments, one skilled in the art will recognize that numerous modifications are possible. Embodiments of the disclosure can be realized using a variety of computer systems and communication technologies, including but not limited to the specific examples described herein. Embodiments of the present disclosure can be realized using any combination of dedicated components or programmable processors or other programmable devices. The various processes described herein can be implemented on the same processor or different processors in any combination. Where components are described as being configured to perform certain operations, such configuration can be accomplished, e.g., by designing electronic circuits to perform the operation, by programming programmable electronic circuits (such as microprocessors) to perform the operation, or any combination thereof. Further, while the embodiments described above may make reference to specific hardware and software components, those skilled in the art will appreciate that different combinations of hardware or software components may also be used and that particular operations described as being implemented in hardware might also be implemented in software or vice versa.

Computer programs incorporating various features of the present disclosure may be encoded and stored on various computer-readable storage media; suitable media include magnetic disk or tape, optical storage media such as compact disk (CD) or digital versatile disk (DVD), flash memory, and other non-transitory media. Computer-readable media encoded with the program code may be packaged with a compatible electronic device, or the program code may be provided separately from electronic devices (e.g., via Internet download or as a separately packaged computer-readable storage medium).

Thus, although the disclosure has been described with respect to specific embodiments, it will be appreciated that the disclosure is intended to cover all modifications and equivalents within the scope of the following claims.

Aspects of the present disclosure are set out in the following numbered clauses, in which:
1. A method of providing instructions for activities to address maladaptive behaviors associated with substance use in users, comprising:
   identifying, by one or more processors, for a user with a substance use disorder, a profile including a plurality of substance use words;
   presenting, by the one or more processors, a plurality of memory words not related to the substance use, and an instruction to memorize the plurality of memory words;
   providing, by the one or more processors, an instruction to perform an activity to refrain from selecting one or more of the plurality of memory words and/or one or more of the plurality of substance use words; and
   updating, by the one or more processors, the profile of the user using the performance of the user in the activity.
2. The method of clause 1, further comprising:
   providing, by the one or more processors, an instruction to perform an activity to provide information on a future event.
3. The method of clause 2, wherein the future event is related to at least one of the user's health, relationships, values, finances, enjoyment, charitable contributions, sobriety, small steps, achievement, future gratitude, challenges, lifestyle, or overcoming maladaptive behavior associated with substance use.
4. The method of clause 3, further comprising:
   providing, by the one or more processors, prior to providing the instruction to perform the activity, an instruction for the user to select a type of future event selected from at least one of the user's health, relationships, values, finances, enjoyment, charitable contributions, sobriety, small steps, achievement, future gratitude, challenges, lifestyle, or overcoming maladaptive behavior associated with substance use.
5. The method of clause 2, further comprising:
   providing, by the one or more processors, prior to the instruction to perform the activity, an instruction for the user to select a category of the future event.
6. The method of clause 5, wherein the category of the future event is selected from the user's health, relationships, values, finances, enjoyment, charitable contributions, sobriety, small steps, achievement, future gratitude, challenges, lifestyle, or overcoming maladaptive behavior associated with substance use.
7. The method of clause 2, further comprising:
   providing, by the one or more processors, an instruction to select a timeframe for the future event.
8. The method of clause 7, wherein the future event may occur in less than a month.
9. The method of clause 7, wherein the future event may occur in more than a month.
10. The method of clause 2, further comprising:
   instructing the user to generate one or more visual cues based on the information on the future event provided by the user.
11. The method of clause 10, wherein the visual cue is an image or text, including free text provided by the user.
12. The method of any one of clauses 1 to 11, further comprising:
   providing, by the one or more processors, a subsequent instruction to perform an activity to refrain from selecting one or more of the plurality of memory words and/or one or more of the plurality of substance use words; and
   providing, by the one or more processors, a prompt containing the visual cue related to the information on the future event and an instruction to perform an activity related to the visual cue.
13. The method of clause 12, wherein, the prompt is a timeline identifying more than one visual cues.
14. The method of clause 12, wherein the timeline automatically updates based on the time tagged to the visual cue.
15. The method of clause 12, wherein the activity includes an audio recording or a textual description.
16. The method of any one of clauses 2-15, further comprising:
   providing, by the one or more processors, following performance of the activity to provide information, a prompt related to the information on the future event.
17. The method of any one of clauses 2-16, wherein the activity to refrain from selecting a presentation of one or more of the plurality of memory words is performed concurrently with the activity to refrain from selecting one or more of the plurality of substance use words.
18. The method of any one of clauses 1-17, further comprising:
   providing, by the one or more processors, a subsequent instruction to perform an activity to refrain from selecting one or more of the plurality of memory words and/or to refrain from selecting one or more of the plurality of substance use words.
19. The method of any one of clauses 16-18, further comprising:
   providing, by the one or more processors, a subsequent instruction to perform an activity to refrain from selecting one or more of the plurality of memory words and/or one or more of the plurality of substance use words; and
   providing, by the one or more processors, following performance of the activity to refrain from selecting one or more of the plurality of memory words and presentation of one or more of the plurality of substance use words, the prompt related to the information on the future event.
20. The method of any one of clauses 1-19, further comprising:
   providing, by the one or more processors, an instruction to perform an activity to select all of the memory words from a plurality of words.
21. The method of any one of clauses 1-20, further comprising:
   providing, by the one or more processors, an instruction to perform an activity to select one or more of the memory words from a plurality of words.
22. The method of clause 20 or clause 21, further comprising:
   determining, by the one or more processors, a score indicating a percentage of correct interactions with the memory words; and
   selecting, by the one or more processors, the plurality of memory words based on the score.
23. The method of any one of clauses 1-22, further comprising:
   determining, by the one or more processors, a score indicating correct interactions, speed of selections, or both during performance of one or more activities; and
   selecting, by the one or more processors, a subsequent plurality of substance use words or a subsequent plurality of memory words, or both based on the score.
24. The method of clause 23, wherein the score is determined by the number of correct interactions, speed of selections, or both during performance of the activity to select all the memory words from a plurality of words.
25. The method of any one of clauses 1-24, further comprising:
   determining, by the one or more processors, a score indicating correct interactions, speed of the selections, or both during performance of the activity to refrain from selecting the plurality of memory words and/or the plurality of substance use words; and
   determining, by the one or more processors, responsive to the score satisfying a threshold, an increase in a number of words for a subsequent activity to refrain from selecting the plurality of memory words and/or the plurality of substance use words.
26. The method of any one of clauses 1-24, further comprising:
   determining, by the one or more processors, a score indicating correct interactions, speed of the selections, or both during performance of the activity to select all memory words from a plurality of words; and
   determining, by the one or more processors, responsive to the score satisfying a threshold, an increase in a number of words for a subsequent activity to refrain from selecting a plurality of memory words, an activity to refrain from selecting a plurality of substance use words, or an activity to select all memory words from a plurality of words.
27. The method of any one of clauses 22-26, further comprising:
   displaying the score indicating at least one of a percentage of correct interactions, speed of selections, or both during one or more activities.
28. The method of any one of clauses 2-27, further comprising:
   generating, by the one or more processors, one or more cue words or images based on the information on the future event provided by the user; and
   wherein the prompt related to the information on the future event comprises the one or more cue words or images.
29. The method of any one of clauses 1-28, wherein providing the instruction to perform the activity to refrain from selecting one or more of the plurality of substance use words and/or the one or more of the plurality of memory words further comprises providing, by the one or more processors, an instruction to perform an activity for the user to select one or more of a plurality of neutral words unrelated to substance use and different than the memory words.
30. The method of any one of clauses 1-29, wherein providing the instruction to perform an activity to refrain from selecting one or more of the plurality of substance use words or memory words comprises presenting the one or more of the plurality of words for less than 1 second, less than 2 seconds, less than 3 seconds, 5 seconds, or equal to or greater than 10 seconds.
31. The method of any one of clauses 1-30, wherein the instruction to perform an activity is provided to the user at least once a day, at least once every 2 days, at least once every 5 days, or at least once a week.
32. The method of any one of clauses 1-31, wherein the user is administered an effective amount of a medication comprising at least one of naloxone, naltrexone, nalmefene, samidorphan, nalorpine, levallorphan, nalodeine, alvimopan, methylnatrexone, naloxegol, acamprosate, disulfiram, topiramate, gabapentin, methadone, buprenorphine, or lofexidine, or a GLP-1 agonist.
33. The method of any one of clauses 1-32, further comprising:
   receiving, by the one or more processors, a request from the user indicating an onset of the behavior associated with the substance use; and
   initiating, by the one or more processors, responsive to receiving the request, the instructions for activities to address maladaptive behaviors associated with substance use in the user.
34. The method of any one of clauses 1-33, further comprising:
   determining, by the one or more processors, a time at which to provide the instruction to perform the activity to refrain from selecting one or more of the plurality of memory words and/or the plurality of substance use words, and
   wherein providing the instruction to perform the activity to refrain from selecting one or more of the plurality of substance use words and/or the plurality of memory words further comprises providing the instruction at the determined time.
35. The method of any one of clauses 1-34, wherein the one or more activities is performed over a period of 1 minute, 2 minutes, 3 minutes, 5 minutes, 7 minutes, 10 minutes or longer.
36. The method of any one of clauses 1-35, wherein the substance use comprises at least one of alcohol abuse disorder (AUD), opioid abuse disorder (OUD), or drug abuse.
37. The method of any one of clauses 1-36, wherein the prompt is provided following at least 1 day, 5 days, 1 week, or one month following the providing of the instruction to perform an activity to provide information on a future event.
38. A system of providing instructions for activities to address maladaptive behaviors associated with substance use in users, comprising:
   one or more processors configured to:
   identify, for a user with a substance use disorder, a profile including a plurality of substance use words;
   present a plurality of memory words not related to the substance use, and an instruction to memorize the plurality of memory words;
   provide an instruction to perform an activity to refrain from selecting one or more of the plurality of memory words and/or one or more of the plurality of substance use words; and
   update the profile of the user using the performance of the user in the activity.
39. The system of clause 38, wherein the one or more processors further configured to:
   provide an instruction to perform an activity to provide information on a future event.
40. The system of clause 39, wherein the future event is related to at least one of the user's health, relationships, values, finances, enjoyment, charitable contributions, sobriety, small steps, achievement, future gratitude, challenges, lifestyle, or overcoming maladaptive behavior associated with substance use.
41. The system of clause 40, wherein the one or more processors is further configured to:
   provide prior to providing the instruction to perform the activity, an instruction for the user to select a type of future event selected from at least one of the user's health, relationships, values, finances, enjoyment, charitable contributions, sobriety, small steps, achievement, future gratitude, challenges, lifestyle, or overcoming maladaptive behavior associated with substance use.
42. The system of clause 39, wherein the one or more processors further configured to:
   provide, by the one or more processors, prior to the instruction to perform the activity, an instruction for the user to select a category of the future event.
43. The system of clause 42, wherein the category of the future event is selected from the user's health, relationships, values, finances, enjoyment, charitable contributions, sobriety, small steps, achievement, future gratitude, challenges, lifestyle, or overcoming maladaptive behavior associated with substance use.
44. The system of clause 39, wherein the one or more processors further configured to:
   provide, by the one or more processors, an instruction to select a timeframe for the future event.
45. The system of clause 44, wherein the future event may occur in less than a month.
46. The system of clause 44, wherein the future event may occur in more than a month.
47. The system of clause 39, wherein the one or more processors further configured to:
   instruct the user to generate one or more visual cues based on the information on the future event provided by the user.
48. The system of clause 47, wherein the visual cue is an image or text, including free text provided by the user.
49. The system of any one of clauses 38-48, wherein the one or more processors further configured to:
   provide a subsequent instruction to perform an activity to refrain from selecting one or more of the plurality of memory words and/or one or more of the plurality of substance use words; and
   provide a prompt containing the visual cue related to the information on the future event and an instruction to perform an activity related to the visual cue.
50. The system of clause 49, wherein, the prompt is a timeline identifying more than one visual cues.
51. The system of clause 49, wherein the timeline automatically updates based on the time tagged to the visual cue.
52. The system of clause 49, wherein the activity includes an audio recording or a textual description.
53. The system of any one of clauses 39-52, wherein the one or more processors further configured to:
   provide following performance of the activity to provide information, a prompt related to the information on the future event.
54. The system of any one of clauses 38-53, wherein the activity to refrain from selecting one or more of the plurality of memory words is performed concurrently with the activity to refrain from selecting one or more of the plurality of substance use words.
55. The system of any one of clauses 38-54, wherein the one or more processors further configured to:
   provide a subsequent instruction to perform an activity to refrain from selecting one or more of the plurality of memory words and/or to refrain from selecting one or more of the plurality of substance use words.
56. The system of any one of clauses 53-55, wherein the one or more processors further configured to:
   provide a subsequent instruction to perform an activity to refrain from selecting one or more of the plurality of memory words and/or one or more of the plurality of substance use words; and
   provide, following performance of the activity to refrain from selecting one or more of the plurality of memory words and presentation of one or more of the plurality of substance use words, the prompt related to the information on the future event.
57. The system of any one of clauses 38-56, wherein the one or more processors further configured to:
   provide an instruction to perform an activity to select all of the memory words from a plurality of words.
58. The system of any one of clauses 38-57, wherein the one or more processors further configured to:
   provide an instruction to perform an activity to select one or more of the memory words from a plurality of words.
59. The system of clause 57 or clause 58, wherein the one or more processors further configured to:
   determine a score indicating a percentage of correct interactions with the memory words; and
   select the plurality of memory words based on the score.
60. The system of any one of clauses 38-59, wherein the one or more processors further configured to:
   determine a score indicating correct interactions, speed of selections, or both during performance of one or more activities; and
   select a subsequent plurality of substance use words or a subsequent plurality of memory words, or both based on the score.
61. The system of clause 60, wherein the score is determined by the number of correct interactions, speed of selections, or both during performance of the activity to select all the memory words from a plurality of words.
62. The system of any one of clauses 38-61, wherein the one or more processors further configured to:
   determine a score indicating correct interactions, speed of the selections, or both during performance of the activity to refrain from selecting the plurality of memory words and/or the plurality of substance use words; and
   determine responsive to the score satisfying a threshold, an increase in a number of words for a subsequent activity to refrain from selecting the plurality of memory words and/or the plurality of substance use words.
63. The system of any one of clauses 38-62, wherein the one or more processors further configured to:
   determine a score indicating correct interactions, speed of the selections, or both during performance of the activity to select all memory words from a plurality of words; and
   determine, responsive to the score satisfying a threshold, an increase in a number of words for a subsequent activity to refrain from selecting a plurality of memory words, an activity to refrain from selecting a plurality of substance use words, or an activity to select all memory words from a plurality of words.
64. The system of any one of clauses 59-63, wherein the one or more processors further configured to:
   display the score indicating at least one of a percentage of correct interactions, speed of selections, or both during one or more activities.
65. The system of any one of clauses 38-64, wherein the one or more processors further configured to:
   generate one or more cue words or images based on the information on the future event provided by the user; and
   wherein the prompt related to the information on the future event comprises the one or more cue words or images.
66. The system of any one of clauses 38-65, wherein providing the instruction to perform the activity to refrain from selecting one or more of the plurality of substance use words and/or the one or more of the plurality of memory words further comprises providing, by the one or more processors, an instruction to perform an activity for the user to select one or more of a plurality of neutral words unrelated to substance use and different than the memory words.
67. The system of any one of clauses 38-66, wherein providing the instruction to perform an activity to refrain from selecting one or more of the plurality of substance use words and/or memory words comprises presenting the one or more of the plurality of words for less than 1 second, less than 2 seconds, less than 3 seconds, 5 seconds, or equal to or greater than 10 seconds.
68. The system of any one of clauses 38-67, wherein the instruction to perform an activity is provided to the user at least once a day, at least once every 2 days, at least once every 5 days, or at least once a week.
69. The system of any one of clauses 38-68, wherein the user is administered an effective amount of a medication comprising at least one of naloxone, naltrexone, nalmefene, samidorphan, nalorpine, levallorphan, nalodeine, alvimopan, methylnatrexone, naloxegol, acamprosate, disulfiram, topiramate, gabapentin, methadone, buprenorphine, or lofexidine, or a glp-1 agonist.
70. The system of any one of clauses 38-69, wherein the one or more processors further configured to:
   receive a request from the user indicating an onset of the behavior associated with the substance use; and
   initiate responsive to receiving the request, the instructions for activities to address maladaptive behaviors associated with substance use in the user.
71. The system of any one of clauses 38-10, wherein the one or more processors further configured to:
   determine a time at which to provide the instruction to perform the activity to refrain from selecting one or more of the plurality of memory words and/or the plurality of substance use words, and
   wherein providing the instruction to perform the activity to refrain from selecting one or more of the plurality of substance use words and/or the plurality of memory words further comprises providing the instruction at the determined time.
72. The system of any one of clauses 38-71, wherein the one or more activities is performed over a period of 1 minute, 2 minutes, 3 minutes, 5 minutes, 7 minutes, 10 minutes or longer.
73. The system of any one of clauses 38-72, wherein the substance use comprises at least one of alcohol abuse disorder (AUD), opioid abuse disorder (OUD), or drug abuse.
74. The system of any one of clauses 38-73, wherein the prompt is provided following at least 1 day, 5 days, 1 week, or one month following the providing of the instruction to perform an activity to provide information on a future event.
75. A method of treating or ameliorating substance use disorder (SUD) in a user in need thereof, comprising:
   administering to the user a treatment comprising a digital therapeutic, the digital therapeutic comprising:
   identifying for a user with a substance use disorder, a profile including a plurality of substance use words;
   presenting a plurality of memory words not related to the substance use, and an instruction to memorize the plurality of memory words; and
   providing an instruction to perform an activity to refrain from selecting one or more of the plurality of memory words and/or one or more of the plurality of substance use words.
76. The method of clause 75, wherein administering the digital therapeutic further comprises:
   providing an instruction to perform an activity to provide information on a future event.
77. The method of clause 76, wherein the future event is related to at least one of the user's health, relationships, values, finances, enjoyment, charitable contributions, sobriety, small steps, achievement, future gratitude, challenges, lifestyle, or overcoming maladaptive behavior associated with substance use.
78. The method of clause 77, wherein administering the digital therapeutic further comprises:
   providing prior to providing the instruction to perform the activity, an instruction for the user to select a type of future event selected from at least one of the user's health, relationships, values, finances, enjoyment, charitable contributions, sobriety, small steps, achievement, future gratitude, challenges, lifestyle, or overcoming maladaptive behavior associated with substance use.
79. The method of clause 76, wherein administering the digital therapeutic further comprises:
   providing prior to the instruction to perform the activity, an instruction for the user to select a category of the future event.
80. The method of clause 79, wherein the category of the future event is selected from the user's health, relationships, values, finances, enjoyment, charitable contributions, sobriety, small steps, achievement, future gratitude, challenges, lifestyle, or overcoming maladaptive behavior associated with substance use.
81. The method of clause 76, wherein administering the digital therapeutic further comprises:
   providing an instruction to select a timeframe for the future event.
82. The method of clause 81, wherein the future event may occur in less than a month.
83. The method of clause 81, wherein the future event may occur in more than a month.
84. The method of clause 76, wherein administering the digital therapeutic further comprises:
   instructing the user to generate one or more visual cues based on the information on the future event provided by the user.
85. The method of clause 84, wherein the one or more visual cues is an image or text, including free text provided by the user.
86. The method of any one of clauses 75-85, wherein administering the digital therapeutic further comprises:
   providing a subsequent instruction to perform an activity to refrain from selecting one or more of the plurality of memory words and/or one or more of the plurality of substance use words; and
   providing a prompt including the one or more visual cues related to the information on the future event and an instruction to perform an activity related to the one or more visual cues.
87. The method of clause 86, wherein, the prompt is a timeline identifying more than one visual cues.
88. The method of clause 87, wherein the timeline automatically updates based on the time tagged to the visual cue.
89. The method of clause 86, wherein the activity includes an audio recording or a textual description.
90. The method of any one of clauses 78-89, wherein administering the digital therapeutic further comprises:
   providing following performance of the activity to provide information, a prompt related to the information on the future event.
91. The method of any one of clauses 75-90, wherein the activity to refrain from selecting one or more of the plurality of memory words is performed concurrently with the activity to refrain from selecting one or more of the plurality of substance use words.
92. The method of any one of clauses 75-91, wherein administering the digital therapeutic further comprises:
   providing a subsequent instruction to perform an activity to refrain from selecting one or more of the plurality of memory words and/or to refrain from selecting one or more of the plurality of substance use words.
93. The method of any one of clauses 90-92, wherein administering the digital therapeutic further comprises:
   providing a subsequent instruction to perform an activity to refrain from selecting one or more of the plurality of memory words and/or one or more of the plurality of substance use words; and
   providing following performance of the activity to refrain from selecting one or more of the plurality of memory words and presentation of one or more of the plurality of substance use words, the prompt related to the information on the future event.
94. The method of any one of clauses 75-93, wherein administering the digital therapeutic further comprises:
   providing an instruction to perform an activity to select all of the memory words from a plurality of words.
95. The method of any one of clauses 75-94, wherein administering the digital therapeutic further comprises:
   providing an instruction to perform an activity to select one or more of the memory words from a plurality of words.
96. The method of clause 94 or clause 95, wherein administering the digital therapeutic further comprises:
   determining a score indicating a percentage of correct interactions with the memory words; and
   selecting the plurality of memory words based on the score.
97. The method of any one of clauses 75-96, wherein administering the digital therapeutic further comprises:
   determining a score indicating correct interactions, speed of selections, or both during performance of one or more activities; and
   selecting a subsequent plurality of substance use words or a subsequent plurality of memory words, or both based on the score.
98. The method of clause 97, wherein the score is determined by a number of correct interactions, speed of selections, or both during performance of the activity to select all the memory words from a plurality of words.
99. The method of any one of clauses 75-95, wherein administering the digital therapeutic further comprises:
   determining a score indicating correct interactions, speed of the selections, or both during performance of the activity to refrain from selecting the plurality of memory words and/or the plurality of substance use words; and
   determining, responsive to the score satisfying a threshold, an increase in a number of words for a subsequent activity to refrain from selecting the plurality of memory words and/or the plurality of substance use words.
100. The method of any one of clauses 94-97, wherein administering the digital therapeutic further comprises:
   determining a score indicating correct interactions, speed of the selections, or both during performance of the activity to select all memory words from a plurality of words; and
   determining, responsive to the score satisfying a threshold, an increase in a number of words for a subsequent activity to refrain from selecting a plurality of memory words, an activity to refrain from selecting a plurality of substance use words, or an activity to select all memory words from a plurality of words.
101. The method of any one of clauses 96-100, wherein administering the digital therapeutic further comprises:
   displaying the score indicating at least one of a percentage of correct interactions, speed of selections, or both during one or more activities.
102. The method of any one of clauses 76-101, further comprising:
   Generating one or more cue words or images based on the information on the future event provided by the user; and
   wherein the prompt related to the information on the future event comprises the one or more cue words or images.
103. The method of any one of clauses 75-102, wherein providing the instruction to perform the activity to refrain from selecting one or more of the plurality of substance use words and/or the one or more of the plurality of memory words further comprises providing an instruction to perform an activity for the user to select one or more of a plurality of neutral words unrelated to substance use and different than the memory words.
104. The method of any one of clauses 75-103, wherein providing the instruction to perform an activity to refrain from selecting one or more of the plurality of substance use words or memory words comprises presenting the one or more of the plurality of words for less than 1 second, less than 2 seconds, less than 3 seconds, 5 seconds, or equal to or greater than 10 seconds.
105. The method of any one of clauses 75-104, wherein the instruction to perform an activity is provided to the user at least once a day, at least once every 2 days, at least once every 5 days, or at least once a week.
106. The method of any one of clauses 75-105, further comprising administering an effective amount of a medication comprising at least one of naloxone, naltrexone, nalmefene, samidorphan, nalorpine, levallorphan, nalodeine, alvimopan, methylnatrexone, naloxegol, acamprosate, disulfiram, topiramate, gabapentin, methadone, buprenorphine, or lofexidine, or a GLP-1 agonist.
107. The method of any one of clauses 75-106, wherein administering the digital therapeutic further comprises:
   receiving a request from the user indicating an onset of a behavior associated with the substance use; and
   initiating responsive to receiving the request, the instructions for activities to address maladaptive behaviors associated with substance use in the user.
108. The method of any one of clauses 75-107, wherein administering the digital therapeutic further comprises:
   determining a time at which to provide the instruction to perform the activity to refrain from selecting one or more of the plurality of memory words and/or the plurality of substance use words, and
   wherein providing the instruction to perform the activity to refrain from selecting one or more of the plurality of substance use words and/or the plurality of memory words further comprises providing the instruction at the determined time.
109. The method of any one of clauses 75-108, wherein the one or more activities is performed over a period of 1 minute, 2 minutes, 3 minutes, 5 minutes, 7 minutes, 10 minutes or longer.
110. The method of any one of clauses 75-109, wherein the substance use comprises at least one of alcohol abuse disorder (AUD), opioid abuse disorder (OUD), or drug abuse.
111. The method of any one of clauses 75-110, wherein the prompt is provided following at least 1 day, 5 days, 1 week, or one month following the providing of the instruction to perform an activity to provide information on a future event.
112. The method of any one of clauses 75-111, wherein the SUD in the user is ameliorated when the user experiences a decrease in Obsessive Compulsive Drug Use Scale (OCDUS) metric following administration of the digital therapeutic.
113. The method of any one of clauses 75-112, wherein the SUD in the user is ameliorated when the user experiences an improvement in a Drug Abstinence Self-Efficacy Scale (DASES) metric following administration of the digital therapeutic.
114. The method of any one of clauses 75-113, wherein the SUD in the user is ameliorated when the user experiences an improvement in a Modified Visual Analogue Scale (MVAS) rating for cravings following administration of the digital therapeutic.
115. The method of any one of clauses 75-114, wherein the SUD in the user is ameliorated when the user experiences a decrease in self-reported substance use following administration of the digital therapeutic.
116. The method of any one of clauses 75-115, wherein the SUD in the user is ameliorated when the user experiences an improvement in an Opioid Substitution Treatment Quality of Life (OSTQOL) scale following administration of the digital therapeutic.
117. The method of any one of clauses 75-116, wherein the user is in treatment for substance use, at least in partial concurrence with the administration of the digital therapeutic.
118. The method of any one of clauses 75-117, wherein the user is prescribed a medication for substance use.
119. The method of any one of clauses 75-118, wherein substance use is decreased in the user following administration of the digital therapeutic.
120. The method of any one of clauses 75-119, wherein the SUD in the user is ameliorated when the user exhibits an improvement in at least one of a Recovery Empowerment Scale, Perceived Stress Scale, OSTQOL, MVAS, or self-reported total substance use with the timeline follow-back (TLFB) procedure following administration of the digital therapeutic.
121. The method of any one of clauses 75-120, wherein the digital therapeutic is administered at least daily, every 2 days, or weekly.
122. The method of clause 121, wherein the digital therapeutic is administered for at least one week, one month, or 3 months.
123. The method of any one of clauses 75-122, further comprising:
   updating the profile of the user using the performance of the user in the activity.

## Claims

1. A method of providing instructions for activities to address maladaptive behaviors associated with substance use in users, comprising:
identifying, by one or more processors, for a user with a substance use disorder, a profile including a plurality of substance use words;
presenting, by the one or more processors, a plurality of memory words not related to the substance use, and an instruction to memorize the plurality of memory words;
providing, by the one or more processors, an instruction to perform an activity to refrain from selecting one or more of the plurality of memory words and/or one or more of the plurality of substance use words; and
updating, by the one or more processors, the profile of the user using the performance of the user in the activity.

2. The method of claim 1, further comprising:
providing, by the one or more processors, an instruction to perform an activity to provide information on a future event, and optionally
wherein the future event is related to at least one of the user's health, relationships, values, finances, enjoyment, charitable contributions, sobriety, small steps, achievement, future gratitude, challenges, lifestyle, or overcoming maladaptive behavior associated with substance use, and optionally
the method further comprising:
providing, by the one or more processors, prior to providing the instruction to perform the activity, an instruction for the user to select a type of future event selected from at least one of the user's health, relationships, values, finances, enjoyment, charitable contributions, sobriety, small steps, achievement, future gratitude, challenges, lifestyle, or overcoming maladaptive behavior associated with substance use.

3. The method of claim 2, further comprising:
providing, by the one or more processors, prior to the instruction to perform the activity, an instruction for the user to select a category of the future event, and optionally
wherein the category of the future event is selected from the user's health, relationships, values, finances, enjoyment, charitable contributions, sobriety, small steps, achievement, future gratitude, challenges, lifestyle, or overcoming maladaptive behavior associated with substance use.

4. The method of claim 2, further comprising:
providing, by the one or more processors, an instruction to select a timeframe for the future event, and optionally
wherein the future event may occur in less than a month, or
wherein the future event may occur in more than a month.

5. The method of claim 2, further comprising:
instructing the user to generate one or more visual cues based on the information on the future event provided by the user, and optionally
wherein the visual cue is an image or text, including free text provided by the user.

6. The method of any one of claims 1 to 5, further comprising:
providing, by the one or more processors, a subsequent instruction to perform an activity to refrain from selecting one or more of the plurality of memory words and/or one or more of the plurality of substance use words; and
providing, by the one or more processors, a prompt containing a visual cue related to the information on the future event and an instruction to perform an activity related to the visual cue, and optionally
wherein, the prompt is a timeline identifying more than one visual cues, or
wherein the timeline automatically updates based on the time tagged to the visual cue, or
wherein the activity includes an audio recording or a textual description.

7. The method of any one of claims 2-6, further comprising:
providing, by the one or more processors, following performance of the activity to provide information, a prompt related to the information on the future event.

8. The method of any one of claims 2-7, wherein the activity to refrain from selecting a presentation of one or more of the plurality of memory words is performed concurrently with the activity to refrain from selecting one or more of the plurality of substance use words.

9. The method of any one of claims 1-8, further comprising:
providing, by the one or more processors, a subsequent instruction to perform an activity to refrain from selecting one or more of the plurality of memory words and/or to refrain from selecting one or more of the plurality of substance use words.

10. The method of any one of claims 8 or 9, further comprising:
providing, by the one or more processors, a subsequent instruction to perform an activity to refrain from selecting one or more of the plurality of memory words and/or one or more of the plurality of substance use words; and
providing, by the one or more processors, following performance of the activity to refrain from selecting one or more of the plurality of memory words and presentation of one or more of the plurality of substance use words, the prompt related to the information on the future event.

11. The method of any one of claims 1-10, further comprising:
providing, by the one or more processors, an instruction to perform an activity to select all of the memory words from a plurality of words, and/or
the method further comprising:
providing, by the one or more processors, an instruction to perform an activity to select one or more of the memory words from a plurality of words, and optionally
the method further comprising:
determining, by the one or more processors, a score indicating a percentage of correct interactions with the memory words; and
selecting, by the one or more processors, the plurality of memory words based on the score, and optionally
the method further comprising:
displaying the score indicating at least one of a percentage of correct interactions, speed of selections, or both during one or more activities.

12. The method of any one of claims 1-11, further comprising:
determining, by the one or more processors, a score indicating correct interactions, speed of selections, or both during performance of one or more activities; and
selecting, by the one or more processors, a subsequent plurality of substance use words or a subsequent plurality of memory words, or both based on the score, and optionally
wherein the score is determined by the number of correct interactions, speed of selections, or both during performance of the activity to select all the memory words from a plurality of words.

13. The method of any one of claims 1-12, further comprising:
determining, by the one or more processors, a score indicating correct interactions, speed of the selections, or both during performance of the activity to refrain from selecting the plurality of memory words and/or the plurality of substance use words; and
determining, by the one or more processors, responsive to the score satisfying a threshold, an increase in a number of words for a subsequent activity to refrain from selecting the plurality of memory words and/or the plurality of substance use words.

14. The method of any one of claims 1-13, further comprising:
determining, by the one or more processors, a score indicating correct interactions, speed of the selections, or both during performance of the activity to select all memory words from a plurality of words; and
determining, by the one or more processors, responsive to the score satisfying a threshold, an increase in a number of words for a subsequent activity to refrain from selecting a plurality of memory words, an activity to refrain from selecting a plurality of substance use words, or an activity to select all memory words from a plurality of words.

15. The method of any one of claims 6-14, further comprising:
generating, by the one or more processors, one or more cue words or images based on the information on the future event provided by the user; and
wherein the prompt related to the information on the future event comprises the one or more cue words or images.

16. The method of any one of claims 1-15, wherein providing the instruction to perform the activity to refrain from selecting one or more of the plurality of substance use words and/or the one or more of the plurality of memory words further comprises providing, by the one or more processors, an instruction to perform an activity for the user to select one or more of a plurality of neutral words unrelated to substance use and different than the memory words.

17. The method of any one of claims 1-16, wherein providing the instruction to perform an activity to refrain from selecting one or more of the plurality of substance use words or memory words comprises presenting the one or more of the plurality of words for less than 1 second, less than 2 seconds, less than 3 seconds, 5 seconds, or equal to or greater than 10 seconds.

18. The method of any one of claims 1-17, wherein the instruction to perform an activity is provided to the user at least once a day, at least once every 2 days, at least once every 5 days, or at least once a week and/or

19. The method of any one of claims 1-18, further comprising:
receiving, by the one or more processors, a request from the user indicating an onset of the behavior associated with the substance use; and
initiating, by the one or more processors, responsive to receiving the request, the instructions for activities to address maladaptive behaviors associated with substance use in the user.

20. The method of any one of claims 1-19, further comprising:
determining, by the one or more processors, a time at which to provide the instruction to perform the activity to refrain from selecting one or more of the plurality of memory words and/or the plurality of substance use words, and
wherein providing the instruction to perform the activity to refrain from selecting one or more of the plurality of substance use words and/or the plurality of memory words further comprises providing the instruction at the determined time.

21. The method of any one of claims 1-20, wherein the one or more activities is performed over a period of 1 minute, 2 minutes, 3 minutes, 5 minutes, 7 minutes, 10 minutes or longer.

22. The method of any one of claims 1-21, wherein the substance use comprises at least one of alcohol abuse disorder (AUD), opioid abuse disorder (OUD), or drug abuse.

23. The method of any one of claims 6-22, wherein the prompt is provided following at least 1 day, 5 days, 1 week, or one month following the providing of the instruction to perform an activity to provide information on a future event.

24. A system of providing instructions for activities to address maladaptive behaviors associated with substance use in users, comprising:
one or more processors configured to perform the method of any one preceding claim.
